# EUROPEAN PATENT SPECIFICATION

(11) **EP 2 107 373 B1**
(45) Date of publication and mention of the grant of the patent: **23.05.2012**
(21) Application number: 08703723.0
(22) Date of filing: 23.01.2008
(51) Int. Cl.: G01N 33/53, C12M 1/00, C12N 15/09, C12Q 1/68, G01N 33/543, G01N 37/00

(54) **ANALYSIS CHIP AND ANALYSIS METHOD**
ANALYSECHIP UND ANALYSEVERFAHREN
PUCE D'ANALYSE ET PROCEDE D'ANALYSE

(30) Priority: 24.01.2007 JP 2007013770
(43) Date of publication of application: 07.10.2009
(73) Proprietor: Toray Industries, Inc., Tokyo 103-8666 (JP)
(72) Inventor: NOMURA, Osamu, Kamakura-shi Kanagawa 248-8555 (JP); KURODA, Toshihiko, Kamakura-shi Kanagawa 248-8555 (JP); NOBUMASA, Hitoshi, Kamakura-shi Kanagawa 248-8555 (JP); MURAO, Yasuo, Kamakura-shi Kanagawa 248-0036 (JP)
(74) Representative: Kador & Partner
(86) International application number: PCT/JP2008/050891
(87) International publication number: WO 2008/090922

(56) References cited:
- EP-A1- 1 729 136
- WO-A1-2005/090997
- WO-A1-2006/137334
- US-A- 6 086 780
- ANDREWS E ET AL: "Effect of surfactant layers on the size changes of aerosol particles as a function of relative humidity" ENVIRONMENTAL SCIENCE AND TECHNOLOGY 1993 US, vol. 27, no. 5, 1993, pages 857-865, XP002566936 ISSN: 0013-936X

## Description

### TECHNICAL FIELD

The present invention relates to an analysis chip having a substrate on which a substance capable of selectively binding to a test substance, that is, a selective binding substance, is immobilized, and an analytical method for the test substance using the analysis chip.

### BACKGROUND ART

An analysis chip has a substrate on which a selective binding substance such as a gene, protein, lipid, saccharide or the like is immobilized, which selective binding substance on the substrate is allowed to react with a test substance which is usually in the form of a solution and, from the result of the reaction, presence or absence, condition, or quantity of the test substance is analyzed. Examples of this substrate generally include those made of a glass, metal or resin.

As one.embodiment of an analysis chip, there is an analysis chip called microarray whose substrate has molecules such as DNA deposited thereon at high density for the purpose of assaying expression of hundreds to tens of thousands of numerous genes at the same time. By using microarrays, systematic and exhaustive gene expression analyses can be carried out on various disease animal models and cell biological phenomena. In particular, functions of genes, that is, proteins encoded by the genes can be clarified, and timing of expression of the proteins and places where they act can be identified. It is thought that searching of disease genes and genes related to therapies, and finding of therapeutic methods are possible by analyzing variation of gene expression of organisms at the cell or tissue level by microarrays, and constructing databases for gene expression profiles by combining the resulting data with data on physiological, cell biological and biochemical phenomena.

At present, two basic methods, that is, the Gene Chip method and the cDNA analysis chip method are used for preparation of analysis chips.

The Gene Chip method is a method developed by Affymetrix, wherein oligoDNAs of about 25 mer are synthesized on a glass plate by photolithography, which 25 mer oligoDNAs are designed based on the base sequences from 16 to 20 regions per gene, and the set of the perfectly matching 25 mers and a set of oligomers having a mismatch of one base introduced intentionally by changing the 13th base is used in combination as probe DNAs. Since, in this method, the lengths of the probe DNAs are constant and their sequences are known, GC content which affects hybridization intensity can be made to be constant, so that the above chip is considered to be an ideal analysis chip for a quantitative analysis of expression. On the other hand, the cDNA analysis chip method is a method developed by Stanford University, wherein DNA is immobilized on a glass plate by a method such as the spotting method or the ink-jet method. When an analysis chip prepared by any of these methods is used, a sample (gene) to be measured which was preliminarily fluorescently labeled is allowed to bind to probe DNAs on the analysis chip by hybridization, and its fluorescence intensity is measured by a scanner to assay expression of the gene.

An example of analyses of analysis chip data is hierarchical clustering. By this method, genes having similar expression patterns are collected to prepare a phylogenetic tree, and the expression levels of many genes are schematically represented by different colors. Such clustering enables identification of genes related to certain diseases.

Analysis chips have been more and more used as methods to test and analyze not only nucleic acids such as DNAs but also proteins and saccharides. Especially, in chips for analysis of proteins, proteins such as antibodies, antigens and enzyme substrates are immobilized on the substrate.

When using an analysis chip, it is important to apply a prepared solution containing a test substance such that the solution spreads evenly over the region on the analysis chip where a selective binding substance is immobilized. As means for achieving this, analysis chips having particles therein for stirring the solution containing a test substance are known.

Patent Literature 1 discloses an analysis chip wherein a particle dispersion prepared by preliminarily adding particles in a test substance DNA solution is applied to the analysis chip, which chip is then covered by a cover glass and sealed by a sealing agent, to create a void defined by the cover glass, analysis chip substrate and sealing agent. This analysis chip enables hybridization under stirring using the motion of the particles, without evaporation of the test substance solution.

Patent Literature 2 discloses an analysis chip wherein irregularities are provided on the analysis chip substrate, and a selective binding substance is immobilized on protruded portions of the irregularities and particles for stirring are movably contained in recessed portions thereof, to enable stirring of the reaction solution. Since, in this analysis chip, the particles are kept away from upper surfaces of the protruded portions, stirring with the particles may be carried out without damaging the selective binding substance.

However, in analysis chips wherein stirring is carried out using particles as above, when the solution containing the test substance is applied thereto, bubbles may remain on the surface inside the analysis chip substrate or on the surfaces of the particles, or may be generated in the reaction solution. There has been a problem that the generated bubbles inhibit the reaction between the selective binding substance and the test substance in the areas where the bubbles stay. Furthermore, there has been a problem that unevenness of the reaction between the areas where the bubbles stay and the other areas causes deviation of detection sensitivity or lowering of detection sensitivity.
EP 1 729 136 describes an analysis chip containing particles used for mixing.

Furthermore, when the particles for stirring are contained or injected in the void between the analysis chip substrate and the cover, operation of injection of the particles into the void may be difficult or injection of a sufficient amount of the particles may not be achieved due to electrostatic generation or the like. Furthermore, the injected particles may aggregate and become immobile and, in cases where the test substance solution is injected into the void surrounded by the cover and the substrate in such a condition, the solution does not permeate the areas where the particles aggregate, resulting in entrapping of bubbles, which causes unevenness of the reaction.
Patent Literature 1 JP 3557419 B
Patent Literature 2 WO 2005/090997

### DISCLOSURE OF THE INVENTION

### PROBLEMS TO BE SOLVED BY THE INVENTION

The present invention is directed to resolution of the above problems and provides an analysis chip which suppresses generation of bubbles which inhibit the selective reaction between a test substance and an immobilized selective binding substance, thereby reducing deviation of detection sensitivity and lowering of detection sensitivity caused by unevenness of the reaction. The present invention also provides an analysis chip which prevents aggregation of particles for stirring and simplifies injection of the particles for stirring into the void between the analysis chip substrate and its cover.

### MEANS FOR SOLVING THE PROBLEMS

The present inventors intensively studied to discover that the above problems may be solved by coating the surfaces of the particles for stirring with a surfactant, thereby completing the present invention.

That is, the present invention is an analysis chip comprising: a substrate having a surface on which a selective binding substance(s) is(are) immobilized; a cover member adhered to the substrate; a void between said substrate and said cover member; and particles movably contained or injected in the void; the surfaces of the particles being coated with a surfactant(s).

In one preferred embodiment of the analysis chip of the present invention, the surfactant coated on the surfaces of the particles is an anionic surfactant or a nonionic surfactant.

Further, one preferred embodiment of the analysis chip of the present invention is a substrate comprising an irregular region composed of recessed portions and protruded portions, wherein the selective binding substance(s) is(are) immobilized on upper surfaces of the protruded portions.

Further, in one preferred embodiment of the analysis chip of the present invention, the material constituting the particles coated with the surfactant(s) comprises a ceramic.

Further, in one preferred embodiment of the analysis chip of the present invention, one or more penetrating holes communicating with the void are formed in the cover member.

Further, in one preferred embodiment of the analysis chip of the present invention, the shortest distance between the surface of the substrate, on which the selective binding substance(s) is(are) immobilized, and the cover member is smaller than the diameter of the particles.

Further, in one preferred embodiment of the analysis chip of the present invention, the selective binding substance is a DNA, RNA, protein, peptide, saccharide, sugar chain or lipid.

Further, the present invention is a method for analyzing a test substance, the method comprising the steps of:
bringing the analysis chip of the present invention into contact with a solution containing a test substance, thereby selectively binding the test substance to the selective binding substance immobilized on the surface of the substrate; and
measuring the amount of the test substance bound to the analysis chip through the selective binding substance.

One preferred embodiment of the method of the present invention for analyzing a test substance is a method wherein the solution containing the test substance is subjected to a degassing treatment before bringing the solution containing the test substance into contact with the analysis chip

### EFFECT OF THE INVENTION

According to the analysis chip of the present invention, retention and generation of bubbles in the reaction solution in the analysis chip may be suppressed. As a result, deviation of detection sensitivity and lowering of detection sensitivity due to unevenness of reaction caused by inhibition of the reaction between the selective binding substance and the test substance by the bubbles may be suppressed, thereby allowing detection of the test substance with higher sensitivity.

Further, according to the analysis chip of the present invention, aggregation of particles may be prevented, and injection of the particles into the void between the analysis chip substrate and its cover member may be carried out easily and smoothly.

### BRIEF DESCRIPTION OF THE DRAWINGS

Fig. 1 is a cross-sectional view schematically showing an example of the substrate constituting the analysis chip of the present invention, on which substrate a selective binding substance is immobilized.
Fig. 2 is a cross-sectional view schematically showing an example of generation of a bubble on the surface of the substrate by usage of the substrate in Fig. 1.
Fig. 3 is a perspective view schematically showing an example of the substrate constituting the analysis chip of the present invention, on which substrate a selective binding substance is immobilized.
Fig. 4 is a cross-sectional view schematically showing an example of the substrate in Fig. 3 constituting the analysis chip of the present invention, on which substrate a selective binding substance is immobilized.
Fig. 5 is a longitudinal sectional view schematically showing an example of a jig and a scanner which read results of a reaction using the substrate constituting the analysis chip of the present invention, on which substrate a selective binding substance is immobilized.
Fig. 6 is a perspective view showing an example of penetrating holes and liquid level-halting chambers.
Fig. 7 is a cross-sectional view schematically showing an example of the analysis chip of the present invention.
Fig. 8 is a cross-sectional view schematically showing an example of the analysis chip of the present invention.
Fig. 9 is a cross-sectional view schematically showing an example of the analysis chip of the present invention.
Fig. 10 is a perspective view schematically showing an example of the analysis chip of the present invention having a partition structure.
Fig. 11 is a longitudinal sectional view schematically showing another example of the analysis chip of the present invention having a partition structure.
Fig. 12 is a longitudinal sectional view schematically showing an example of preferred relationships among the irregular region, cover member and particles

### DESCRIPTION OF SYMBOLS

- 1: Substrate
- 2: Particles
- 3A: Protruded portion of cover member
- 3, 3B: Cover member
- 10: Recessed portion
- 11: Protruded portion
- 12: Region on which selective binding substance is immobilized (irregular region)
- 13: Flat area
- 14: Protruded portion of substrate
- 26: Example of generated bubble
- 30, 30C: Adhesive member
- 30A, 30B: Adhesive member for partition structure
- 31: Void or space
- 32: Penetrating hole
- 33: Liquid level-halting chamber
- 34: Sealing member (tape)
- 35: Void between substrate and cover member
- 40: Spring for urging microarray to jig
- 41: Jig
- 42: Abutting surface of jig
- 43: Objective lens
- 44: Laser excitation light
- 45: Selective binding substance immobilized on substrate
- L1: Pitch between protruded portions

### BEST MODE FOR CARRYING OUT THE INVENTION

The analysis chip of the present invention is characterized in that it has a void between a substrate having a surface on which a selective binding substance is immobilized and a cover member adhered to the substrate, in which void particles are movably contained or injected, and that the surfaces of the particles are coated with a surfactant. Coating with a surfactant means that a surfactant is applied or adhered to the surface of the particle or that the surface of the particle is covered with the surfactant, partially or entirely. This coating may be carried out for example by a method described later.

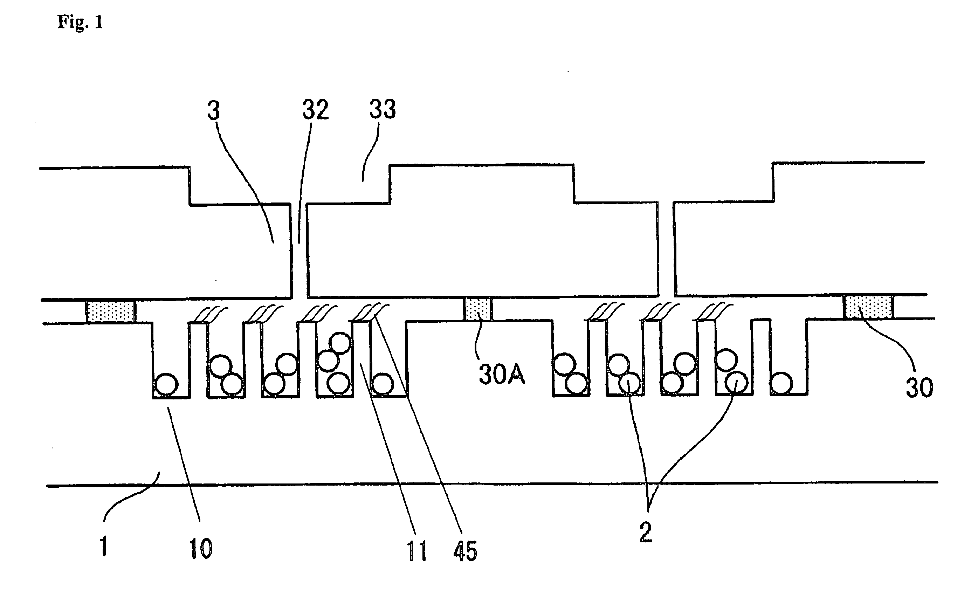

Fig. 1 shows an example of the analysis chip of the present invention containing particles. In the example shown in Fig. 1, the surface of the substrate 1 comprises irregular regions constituted by recessed portions 10 and protruded portions 11. The particles 2 are contained in the recessed portions 10, and the selective binding substance 45 (nucleic acid, for example) is immobilized on the upper surfaces of the protruded portions 11.

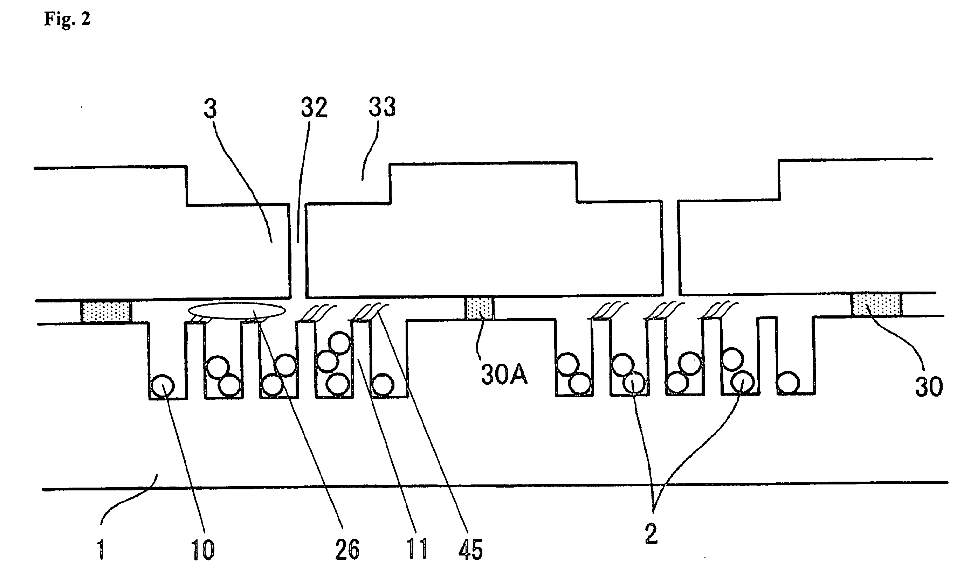

When adding a solution containing a test substance to the analysis chip in order to allow the test substance to react with the selective binding substance 45 (nucleic acid, for example) immobilized on this substrate 1, microbubbles adhered to the surfaces of the particles 2 are liberated into the liquid to form a bubble 26 which covers the protruded portions as shown in Fig. 2, so that the selective binding substance on the surfaces of the covered protruded portions cannot react with the test substance. In the analysis chip of the present invention, by virtue of the fact that the surfaces of the particles 2 are coated with a surfactant, bubbles do not adhere to, or are less likely to adhere to, the surfaces of the particles, thereby enabling suppression of generation of the bubbles. By this, the entire selective binding substances 45 on the surface of the substrate 1 can react with the test substance, and the reliability and reproducibility of obtained data may be increased.

Examples of the method for coating the surfaces of the particles with a surfactant include known methods such as: methods wherein the particles are immersed in a solution containing the surfactant and dried after removal therefrom; methods wherein a solution containing the surfactant is sprayed on the surfaces of the particles, which are then dried; methods wherein the particles are brought into contact with a substance containing a solution of the surfactant; and methods wherein the particles are brought into contact with a liquid, and the surfactant in the form of power is sprinkled thereon. After coating the surfaces of the particles with the surfactant(s) by spraying, adherence or the like using these methods, the particles may be used also after washing away an excess amount of the surfactant with water or an organic solvent. The concentration of the solution containing the surfactant used in these methods is preferably 0.01% to 10%, more preferably 0.05% to 2%.

Examples of the surfactant used for the surface treatment of the particles include anionic surfactants, cationic surfactants, amphoteric surfactants and nonionic surfactants and, among these, anionic surfactants and nonionic surfactants are preferably used.

Examples of the anionic surfactants include sodium dodecyl sulfate (SDS), sodium cholate, sodium deoxycholate, sodium lauryl sarcosinate, polyoxyethylene alkyl ether phosphate, polyoxyethylene alkyl phenyl ether phosphate, triethanolamine lauryl sulfate and sodium lauroyl sarcosinate.

Examples of the cationic surfactants include cetyltrimethylammonium bromide (CTAB), lanolin fatty acid aminopropylethyldimethylammonium ethyl sulfate, alkyltrimethylammonium chloride, dialkyldimethylammonium chloride, distearyldimethylammonium chloride, distearyldimethylbenzylammonium chloride and stearyltrimethylammonium chloride.

Examples of the amphoteric surfactants include 3-[(3-cholamidopropyl)dimethylammonio]-2-hydroxypropanesulfonate (CHAPSO), 3-[(3-cholamidopropyl)dimethylammonio]propanesulfonate (CHAPS), and n-dodecyl-N,N-dimethyl-3-ammonio-1-propanesulfonate (ZWITTERGENT 3-12 Detergent).

Examples of the nonionic surfactants include dimethyldecylphosphine oxide (APO-10), dodecyldimethylphosphine oxide (APO-12), polyoxyethylene lauryl ether (BRIJ-35), polyoxyethylene (20) cetyl ether (BRIJ-58), polyoxyethylene (80) sorbitan monooleate ester (polysorbate 80, Tween 80), polyoxyethylene (20) sorbitan monolaurate ester (polysorbate 20, Tween20), polyethylene glycol p-(1,1,3,3-tetramethylbutyl)phenyl ether (TRITON X-100), TRITON X-114, n-decanoyl-N-methyl-D-glucamine (MEGA-10), n-nonanoyl-N-methyl-D-glucamine (MEGA-9), n-octanoyl-N-methyl-D-glucamine (MEGA-8), nonylphenyl-polyethylene glycol (NP-40), polyoxyethylene polyoxypropylene glycol, ethylene glycol monostearate, sorbitan monostearate, propylene glycol monostearate, polyoxyethylene sorbitan monostearate, polyoxyethylene (160) polyoxypropylene (30) glycol (Pluronic F68), and polyoxyethylene (196) polyoxypropylene (67) glycol (Pluronic F127).

Among these, because of their strong surface-activating effect, sodium dodecyl sulfate (SDS) and sodium deoxycholate are especially preferably used as the anionic surfactant, and Pluronic F68 and Pluronic F127 are especially preferably used as the nonionic surfactant.

The shape of the particle is not restricted as long as it may stir the test substance solution, and the particle may be in an arbitrary shape, for example, a polygon or micro-rod (fine rod) such as a cylinder or prism, in addition to sphere.

The size of the particle is also not restricted, and the diameter of the particle is preferably less than the shortest distance between the surface of the substrate on which the selective binding substance is immobilized and the cover member. For example, in cases where the particle is spherical, its size may be in the range of 0.1 µm to 300 µm. In cases where the particle is cylindrical, the diameter of its bottom surface is regarded as the diameter of the particle, and the diameter of the bottom surface is preferably less than the shortest distance between the surface of the substrate on which the selective binding substance is immobilized and the cover member. For example, in cases where the particle is cylindrical, its length may be in the range of 50 µm to 5000 µm, and the diameter of its bottom surface may be in the range of 10 µm to 300 µm.

The material constituting the particle is also not restricted, and examples thereof include glasses; ceramics (such as yttrium partially-stabilized zirconia); metals and metal oxides such as gold, platinum, stainless, iron, aluminum oxide (alumina) and titanium oxide (titania); and plastics such as nylons and polystyrenes.

The surface of the particle which is coated with the surfactant preferably has an appropriate roughness. That is, the centerline average roughness (Ra value) is preferably not less than 40 nm and not more than 300 nm. By using the particle having the surface roughness in this range, the surface area of the bead is increased, so that the surface of the particle may be coated with more surfactant. In cases where the particle is made of a ceramic, the Ra value is preferably not less than 40 nm and not more than 200 nm in consideration of the strength of the material.

The substrate constituting the analysis chip of the present invention preferably has an irregular region composed of recessed portions and protruded portions, on which protruded portions the selective binding substance is immobilized. Due to such a structure, a nonspecifically-adsorbed test substance is not detected and the noise is reduced in the detection process, thereby results may be obtained with better S/N. A specific reason for the reduction of the noise is as follows. That is, when the substrate wherein the selective binding substance is immobilized on the upper surfaces of the protruded portions is scanned using an apparatus called a scanner, the upper surfaces of the protruded portions are focused by the laser light, thereby the laser light becomes dim at the recessed portions, so that undesirable fluorescence (noise) from the test substance nonspecifically adsorbed to the recessed portions is less likely to be detected.

The heights of the protruded portions in the irregular region are preferably about the same with each other in terms of the heights of the upper surfaces of the protruded portions. Here, the heights are regarded as being about the same in cases where the selective binding substance is immobilized on the surfaces of the protruded portions whose heights vary to a certain extent, which substance is then reacted with the fluorescence-labeled test substance, and scanning is carried out by the scanner, resulting in observation of signals wherein variation of the levels of their intensity does not cause a problem. Specifically, the heights are about the same in cases where the differences among the heights are not more than 50 µm. The differences among the heights are more preferably not more than 30 µm and, still more preferably, the heights are the same. As used herein, "the same height" includes the error due to the variation produced during the process of production or the like. In cases where the difference between the height of the upper surface of the highest protruded portion and the height of the upper surface of the lowest protruded portion is larger than 50 µm, the laser light becomes dim at the upper surfaces of the protruded portions with different heights, and the intensity of the signal from the test substance reacted with the selective binding substance immobilized on these upper surfaces of the protruded portion may be decreased, which is not preferred.

In the substrate constituting the analysis chip of the present invention, the region on which the selective binding substance (nucleic acid, for example) is immobilized is not restricted as long as it is on the surface of the substrate and roughening as described above has not been performed thereto and, in particular, it is preferably the upper surface (upper end surface) of the protruded portion of the irregular region described above. Immobilization of the selective binding substance may be carried out in advance; or only the substrate is prepared without immobilization and, when the test substance is analyzed, the selective binding substance corresponding to the desired test substance may be appropriately selected and immobilized

As the selective binding substance (nucleic acid, for example) which can be immobilized on the upper surfaces of the protruded portions, one necessary for obtaining data may be appropriately selected, but it may also be a mere dummy selective binding substance. It is not necessary to bind the selective binding substance to all the upper surface of the protruded portions, and there may be upper surfaces on which nothing is immobilized.

In the substrate constituting the analysis chip of the present invention, when the selective binding substance(s) is(are) immobilized on the upper surfaces of the protruded portions, the areas of the upper surfaces of the protruded portions are preferably about the same. The upper surfaces of the protruded portions having about the same areas are advantageous for a later analysis since the areas of the regions on which many types of the selective binding substances are immobilized can be made to be the same. Here, the upper surfaces are regarded as having about the same areas in cases where the value obtained by dividing the largest upper surface area among those of the protruded portions by the smallest upper surface area is not more than 1.2.

The area of the upper surface of the protruded portion on which the selective binding substance is immobilized is not restricted and preferably not less than 10 µm² and not more than 1 mm², more preferably not less than 300 µm² and not more than 0.8 mm² from the view point of reducing the amount of the selective binding substance and ease of handling.

In the substrate constituting the analysis chip of the present invention, the surface of the substrate, which surface has the region on which the selective binding substance(s) is(are) immobilized, is preferably surrounded by a flat area having about the same height with the upper end of the protruded portion of the irregular region. Due to such a structure, a solution containing a test substance may be easily applied to the irregular region, and.the particles for stirring may be retrained in the recessed portions without being brought into contact with the selective binding substance(s).

The height of the upper surface of the protruded portion in the irregular region and the height of the flat area are preferably about the same. That is, the difference between the height of the flat area and the height of the upper surfaces of the protruded portions is preferably not more than 50 µm. In cases where the difference between the height of the upper surface of the protruded portion and the height of the flat area is larger than 50 µm, the detectable fluorescence intensity may be lowered, which is not preferred. The difference between the height of the flat area and the height of the upper surface of the protruded portion is more preferably not more than 30 µm and, most preferably, the flat area and the protruded portion have the same height.

The height of the protruded portion in the irregular region of the substrate preferably used in the analysis chip of the present invention, that is, the difference between the height of the upper surface of the protruded portion and the height of the bottom surface of the recessed portion is preferably not less than 10 µm and not more than 500 µm, more preferably not less than 50 µm and not more than 300 µm. In cases where the height of the protruded portion is less than 10 µm, the test substance nonspecifically adsorbed to a region other than the spots may be detected, which results in a poor S/N and is not preferred. In cases where the height of the protruded portion is more than 500 µm, there may be a problem in, for example, that the protruded portion is prone to be broken and damaged, which is not preferred.

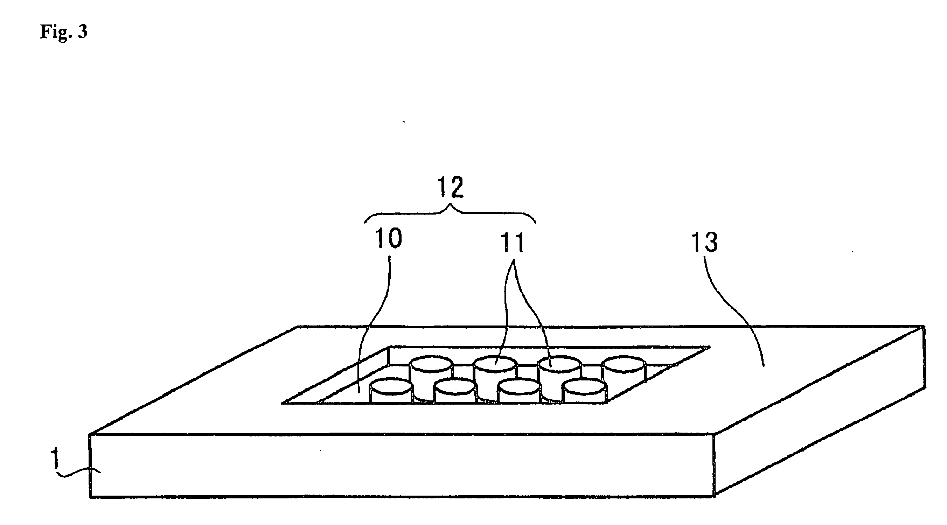

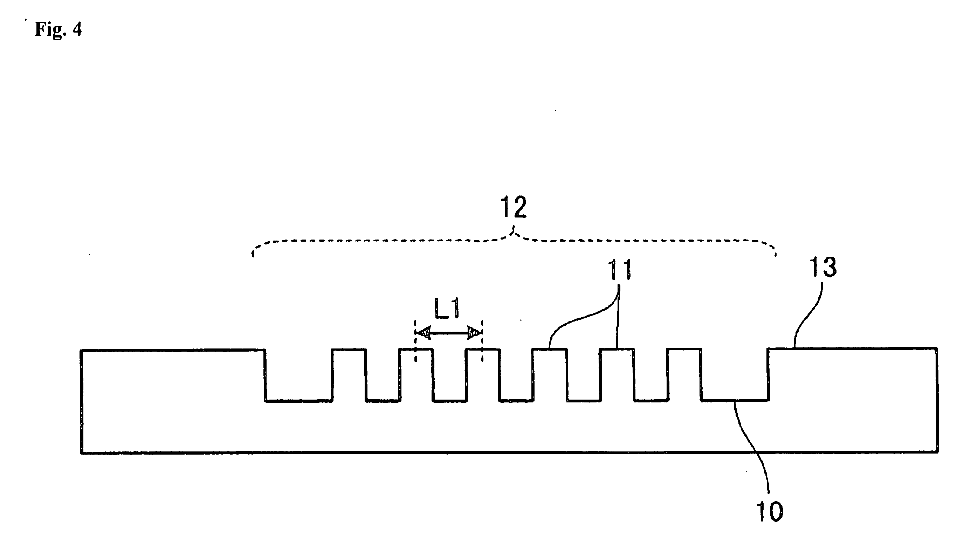

Specific examples of the substrate constituting the analysis chip of the present invention are exemplified in Fig. 3 and Fig. 4.

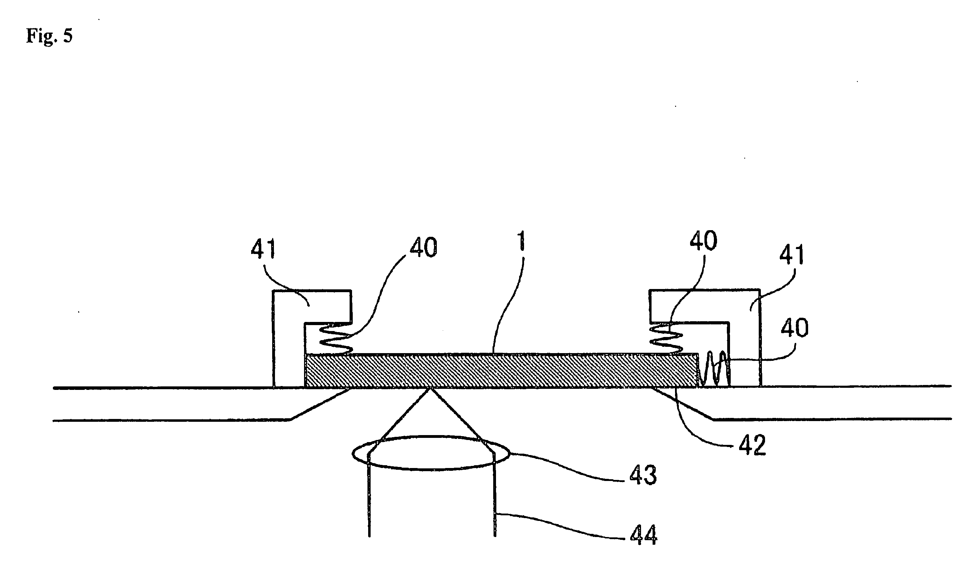

In the examples shown in Fig. 3 and Fig. 4, the surface of the substrate 1 comprises an irregular region 12 comprising multiple protruded potions 11, which irregular region 12 is surrounded by a flat area 13. On the upper surfaces of the protruded portions 11, selective binding substance(s) (nucleic acid, for example) is(are) immobilized. Usage of this flat area enables easy focusing of the measurement light such as an excitation light of a scanner on the upper surface of the protruded portion. More particularly, when the focusing is carried out for radiation of the measurement light such as a laser to the surface of the substrate, as shown in Fig. 5, the substrate 1 is often urged by a spring 40 to a jig 41, and the focus is adjusted in advance by the lens 43 or the like such that the laser light 44 focuses on the height of an abutting surface 42 of the jig. By abutting the flat area of the substrate of the analysis chip of the present invention to the surface 42 of the jig, the measurement light (laser light of the scanner) can be easily focused on the upper surface of the protruded portion of the substrate. In the example shown in Fig. 5, the substrate 1 is fixed such that the surface on which the selective binding substance(s) is(are) immobilized faces downward.

The material which constitutes the substrate of the analysis chip of the present invention is not restricted, and examples thereof include glasses, ceramics, silicone resins, polyethylene terephthalate, cellulose acetate, polycarbonate, polystyrene, polymethyl methacrylate (PMMA), and silicone rubbers such as polydimethylsiloxane (PDMS) elastomers. Among these, polymethyl methacrylate, polystyrene, polydimethylsiloxane (PDMS) elastomers, glasses or silicone resins may be preferably used.

At least a part of the substrate of the analysis chip of the present invention is preferably black. This may reduce autofluorescence from the substrate. The part(s) made to be black may be the main body of the substrate having the irregular region; the side surfaces of the protruded portions; a hydrophobic material or insulating layer provided in the recessed portion; or all of these.

Here, the substrate is regarded as being black in cases where the spectral reflectance of the black portion of the substrate does not show a specific spectral pattern (such as specific peaks) and is uniformly low and the spectral transmittance of the black portion of the substrate also does not show a specific spectral pattern (such as specific peaks) and is uniformly low, within the visible wavelength region (400 nm to 800 nm).

With regard to the values of the spectral reflectance and the spectral transmittance, the spectral reflectance within the visible wavelength region (400 nm to 800 nm) is preferably not more than 7%, and the spectral transmittance within the same wavelength region is preferably not more than 2%. As used herein, the spectral reflectance means a spectral reflectance measured with specular reflection from the substrate using an illumination/light-receiving optical system satisfying the condition C of JIS Z 8722.

The black color of the substrate may be achieved by incorporating a black substance in the substrate of the analysis chip of the present invention. This black substance is not restricted as long as it does not, or is less likely to, reflect light, or it does not, or is less likely to, allow transmission of light, and preferred examples thereof include black substances such as carbon black; graphite; titan black; aniline black; oxides of Ru, Mn, Ni, Cr, Fe, Co or Cu; and carbides of Si, Ti, Ta, Zr or Cr.

These black substances may be incorporated solely or as a mixture of two or more kinds. For example, in the cases of a polymer such as polyethylene terephthalate or a silicone resin, carbon black, graphite, titan black or aniline black among the above black substances may be preferably incorporated, and carbon black may be especially preferably used. In the cases of an inorganic material such as a glass or ceramic, a metal oxide of Ru, Mn, Ni, Cr, Fe, Co, Cu or the like or a carbide of Si, Ti, Ta, Zr or Cr may be preferably incorporated.

The substrate constituting the analysis chip of the present invention may be produced by various methods. For example, in cases where the material is a polymer or the like, the substrate may be molded by a method such as injection molding, hot embossing or a method wherein polymerization is carried out in a mold. In cases where the material is an inorganic material such as a glass or ceramic, the substrate may be molded by sand blasting, and in cases where the material is a silicone resin, it may be molded by a known semiconductor process or the like.

The molded substrate may be subjected to various surface treatments prior to immobilization of the selective binding substance(s) on its surface. Specific examples of such surface treatments include the one described in JP 2004-264289 A.

The analysis chip of the present invention may be used as an analysis chip for analyzing a test substance (sample).

In the present invention, the analysis chip means a chip used for assaying presence or absence of the test substance, the quantity of the test substance, or properties of the test substance, by applying a solution containing the test substance to the chip. Specifically, examples of the analysis chip include biochips wherein a selective binding substance(s) immobilized on the surface of its substrate is allowed to react with a test substance in order to assay the quantity of the test substance or presence or absence of the test substance. More specifically, examples of the analysis chip include DNA chips wherein a nucleic acid is immobilized on the surface of its substrate, protein chips wherein a protein represented by an antibody is immobilized on the surface of its substrate, sugar chain chips wherein a sugar chain is immobilized on the surface of its substrate, cell chips wherein a cell is immobilized on the surface of its substrate.

In the present invention, the selective binding substance means various materials capable of binding selectively to a test substance directly or indirectly. Representative examples of the selective binding substances capable of binding to the surface of the substrate include nucleic acids, proteins, peptides, saccharides and lipids.

The nucleic acid may be a DNA or RNA, and may also be a PNA. Since a single-stranded nucleic acid having a particular base sequence selectively hybridizes with a single-stranded nucleic acid having a base sequence complementary to the base sequence of the nucleic acid or a part thereof, the single-stranded nucleic acid is a selective binding substance in the present invention.

The nucleic acid may be one derived from a natural product such as a live cell or may be one synthesized by a nucleic acid synthesizer. Preparation of DNA or RNA from live cells may be carried out by a known method, for example, for extraction of DNA, the method by Blin et al. (Blin et al., Nucleic Acids Res. 3: 2303 (1976)) or the like, and for extraction of RNA, the method by Favaloro et al. (Favaloro et al., Methods Enzymol.65: 718 (1980)) or the like. Examples of the nucleic acid which may be immobilized further include linear or circular plasmid DNAs and chromosomal DNAs, DNA fragments produced by digestion of these DNAs with a restriction enzyme or by chemical cleavage thereof, DNAs synthesized *in vitro* with an enzyme or the like, or chemically synthesized oligonucleotides.

Examples of the protein include antibodies and antigen-binding fragments of antibodies such as Fab fragment and F(ab')₂ fragment, and various antigens. Since an antibody or an antigen-binding fragment selectively binds to the corresponding antigen, and since an antigen selectively binds to the corresponding antibody, they are "selective binding substances".

Examples of the saccharide include various monosaccharides and sugar chains such as oligosaccharides and polysaccharides.

Examples of the lipid may include simple lipids and complex lipids.

Antigenic substances other than the above nucleic acids, proteins, saccharides and lipids may also be immobilized. Cells may also be immobilized on the surface of the substrate as the selective binding substance.

Among these selective binding substances, those especially preferred are DNAs, RNAs, proteins, peptides, saccharides, sugar chains and lipids.

The analysis chip of the present invention further comprises a cover member covering the surface of the substrate, which cover member is adhered to the substrate. By comprising the cover member, the solution containing the test substance may be easily kept sealed and, as a result, the reaction between the test substance and the selective binding substance(s) immobilized in the region (12 in Fig. 3 or Fig. 4) of the substrate may be stably carried out. The particles may be preliminarily injected (contained) in the analysis chip of the present invention so that the test substance solution may be easily applied. There is also an advantage that the background noise does not increase since the tape and sealing agent do not contact the test substance solution during the operation of closing the penetrating hole after applying the test substance solution.

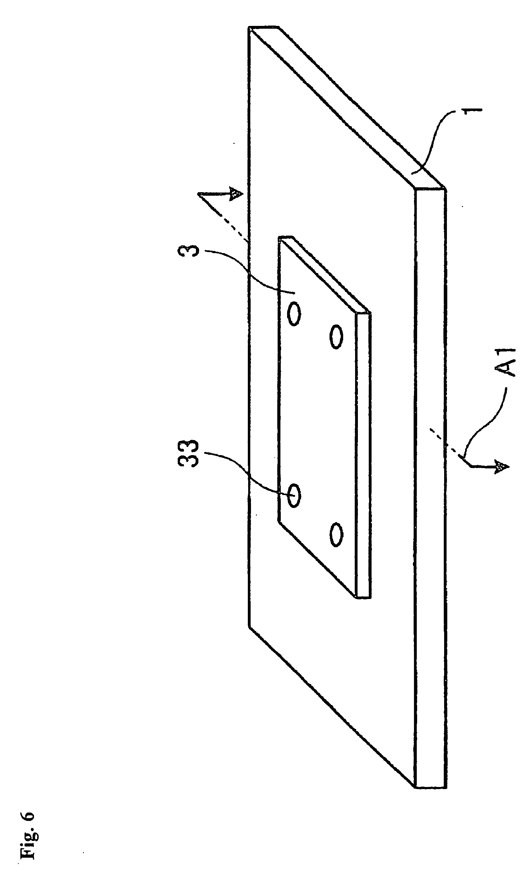

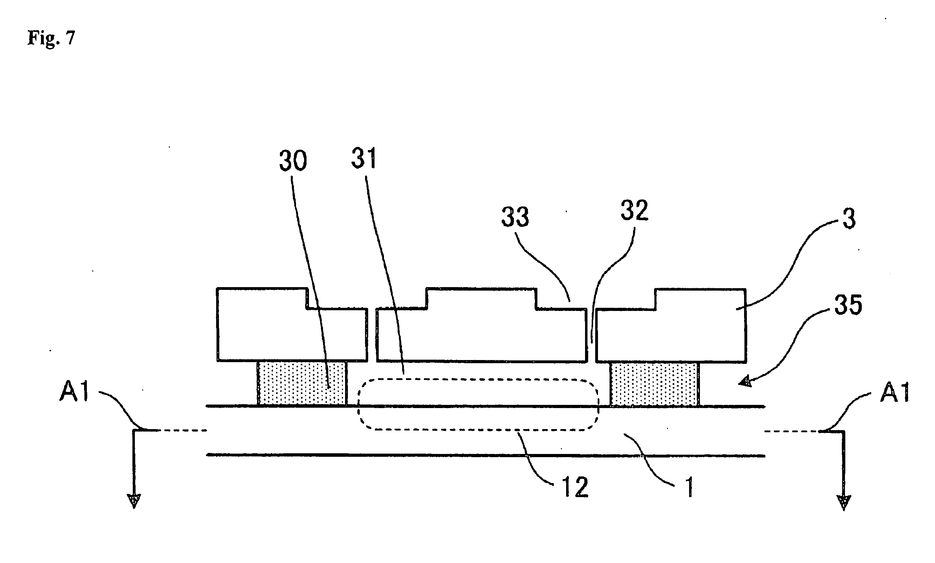

Fig. 6 is a perspective view showing an example of schematic embodiments of the analysis chip of the present invention having, in addition to the substrate, a cover member, adhesive member, penetrating holes and liquid level-halting chambers, and Fig. 7 is a cross-sectional view taken along the plane indicated by the arrow A1 in Fig. 6. In the example shown in Fig. 7, the substrate 1 is covered with a cover member 3 via the adhesive member 30, to form a void 31 comprising the region 12 where the selective binding substance(s) is(are) immobilized. The void 31 is a closed space which does not communicate with the outside except that it communicates with the outside via a plurality of penetrating holes.

The cover member may be adhered such that it covers at least a part of a side of the surface of the substrate and forms a void between the substrate and the cover member. The substrate preferably has a selective binding substance(s) immobilized on a region located in the void, which is the surface of the substrate. That is, the cover member is preferably adhered to the substrate such that the region wherein the selective binding substance(s) is(are) immobilized exists in the void. The cover member may be adhered in any manner as long as the void is formed, and is preferably adhered via an adhesive member such as a double-stick tape or resin composition.

The cover member may comprise one or more penetrating holes communicating with the void, and preferably comprise 2 or more penetrating holes. More specifically, one void preferably has 2 or more penetrating holes, and it especially preferably has 3 to 6 penetrating holes since filling of the solution containing the test substance is simple. As described later, in cases where the void is partitioned into 2 or more spaces which do not communicate with each other, each space preferably has 2 or more, more preferably 3 to 6 penetrating holes. In cases where the cover member has 2 or more penetrating holes, their hole sizes may be the same or different, and in cases where one of the 2 or more penetrating holes is used as an inlet for application of the test substance solution while the other(s) is/are made to function as an air outlet(s), the hole size of the inlet is preferably wide enough to allow application of the solution while the hole size(s) of the other penetrating hole(s) is/are narrower from the view points of simplicity of application of the solution and retention of sealing. Specifically, the diameter of the penetrating hole of the inlet for application is preferably within the range of 0.01 mm to 2.0 mm as described above, and the diameter(s) of the other penetrating hole(s) is/are preferably within the range of 0.01 mm to 1.0 mm.

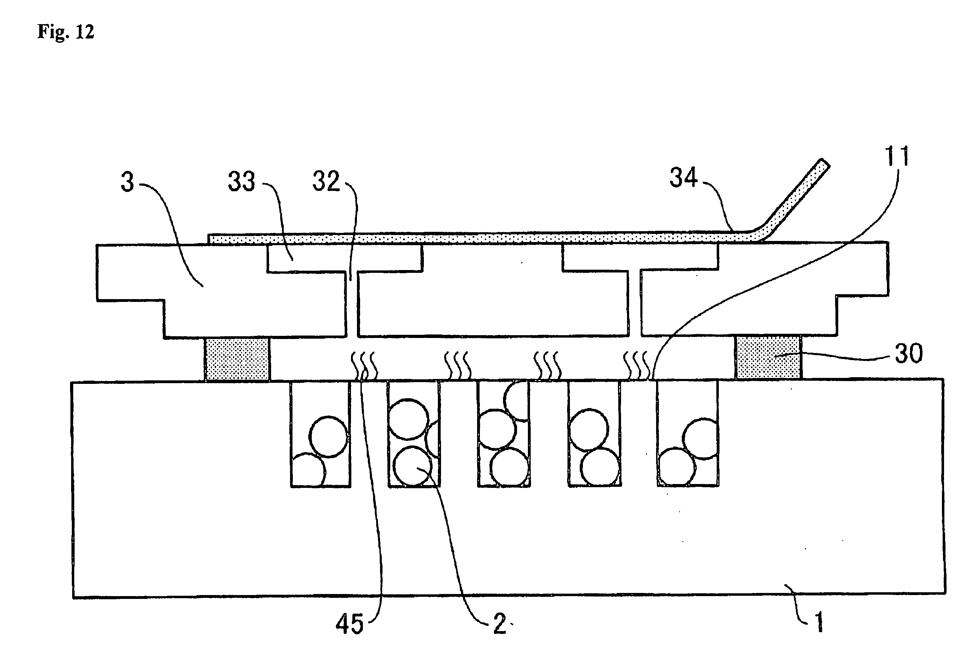

At least one of the penetrating holes 32 may have a different diameter and comprise in its top end a portion with a wider diameter, that is, the liquid level-halting chamber 33. By having the liquid level-halting chamber, rising of the liquid level of the test substance solution applied from the penetrating hole 32 and filled in the void 31 may be suppressed, so that sealing of the penetrating hole with the sealing member 34 (Fig. 12) can be simply and securely carried out and inflow of the air into the test substance solution and outflow of the test substance solution may be prevented, which are preferred. The shape of the liquid level-halting chamber is not restricted, and the chamber may be in a cylindrical, prismatic, conical, pyramidal or hemispherical shape, or in a shape similar thereto. Among these, the cylindrical shape is especially preferred from the view points of simplicity of the production, efficiency of suppressing of the increase of the liquid level of the test substance solution, and the like.

The size of the penetrating hole is not restricted, and in the case of the combination of a cylindrical penetrating hole 32 and a liquid level-halting chamber 33, the hole size (diameter) of the penetrating hole 32 is preferably 0.01 mm to 2.0 mm, more preferably 0.3 mm to 1.0 mm. With a hole size of not less than 0.01 mm, the test substance solution can be easily applied. On the other hand, by making the diameter of the penetrating hole 32 not more than 1.5 mm, evaporation of the test substance solution after application but before sealing and the like may be effectively suppressed. The hole size (diameter) of the liquid level-halting chamber 33 is preferably not less than 1.0 mm. By making the hole size of the liquid level-halting chamber not less than 1.0 mm, a sufficient difference in size relative to the penetrating hole 32 can be obtained, so that a sufficient liquid level-halting effect can be obtained, which is preferred. The upper limit of the diameter of the liquid level-halting chamber is not restricted, and it may be not more than 10 mm. The depth of the liquid level-halting chamber is not restricted, and it may be within the range of 0.1 mm to 5 mm.

Such a cover member is preferably movably adhered to the above described substrate. In cases where the analysis chip of the present invention is used as a DNA chip, it is usually necessary to read the DNA chip using a special scanner, but the chip is difficult to be placed in the special scanner with a cover member adhered thereto, and even when the substrate could be placed in the scanner, the cover member and the optical system component may be made to contact with each other by carrying out a scanning operation, which may result in a trouble. Moreover, even when reading is possible through the cover member, read values may not be accurate. Therefore, the cover member is preferably removable so that the cover member may be removed in the reading step.

The manner in which the cover member is removably adhered to the substrate is not restricted, and an embodiment wherein the cover member may be removed without damaging the cover member and substrate is preferred. For example, the cover member may be adhered via an adhesive member such as a double-stick tape or a resin composition.

When a double-stick tape is used as the adhesive member, a double-stick tape whose both sides show different adhesion strengths is preferably used, and specifically, the surface with the lower adhesion strength is preferably adhered to the substrate side, and the surface with the higher adhesion strength is preferably adhered to the cover member side. With such an embodiment, when the cover member is removed, the double-stick tape and the cover member may be easily removed from the substrate at the same time with the double-stick tape attaching to the cover member, so that inconvenience in the reading step due to the residual adhesive member on the substrate may be avoided. Examples of such a double-stick tape include Product No. 535A produced by Nitto Denko Corporation, Product Nos. 9415PC and 4591HL produced by Sumitomo 3M Limited, and Product No. 7691 produced by Teraoka Seisakusho Co., Ltd.

When a resin composition is used as the adhesive member, examples of the resin composition which may be used include resin compositions comprising a polymer selected from the group consisting of acrylic polymers, silicone polymers and mixtures thereof. Usage of these resin compositions provides improved sealing compared to the double-stick tape and, at the same time, those resin compositions show better stability under a long-term incubation, so that they are especially preferred in an analysis system requiring such a long-term incubation. Especially, in cases where a silicone elastomer is used as the adhesive member, a good sealing performance is provided, and the cover may be adhered such that it may be easily removed. Specific examples of such an elastomer include Sylgard (Sylgard is a registered trademark of Dow Corning) and two-component RTV rubbers (for mold making) produced by Shin-etsu Chemical Co., Ltd.

The shape of the cover member is not restricted as long as it may cover at least a part of a side of the surface of the substrate and form a void between the substrate and the cover member, and may be with a structure around the periphery of the cover, which structure has a part which is more protruded in the portion distant from the substrate than in the portion close to the substrate, that is, an overhang structure. The overhang structure enables easy removal of the cover member without damaging the substrate, which is preferred.

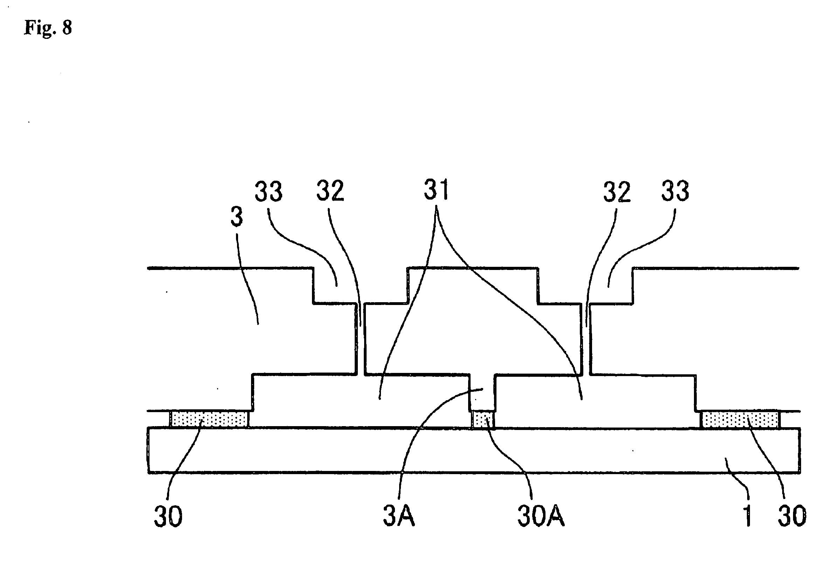

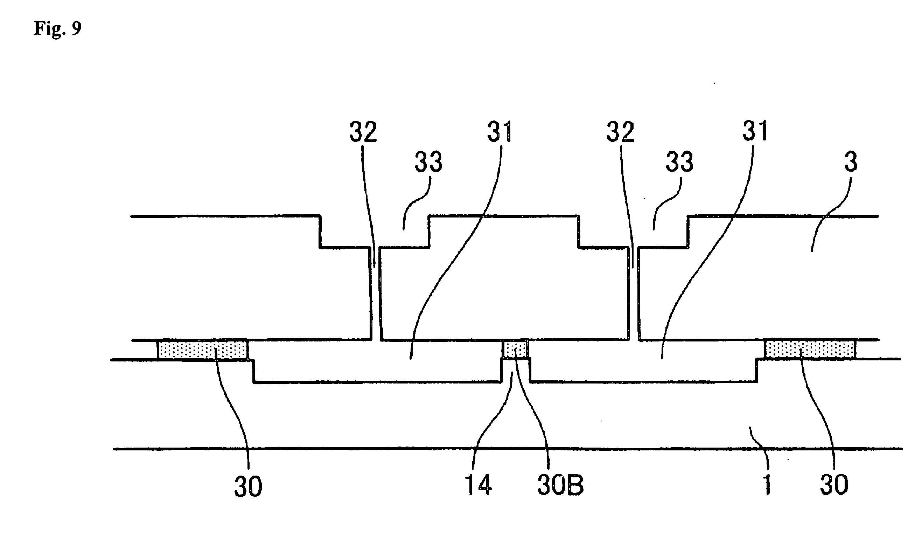

The substrate constituting the analysis chip of the present invention on which substrate the selective binding substance(s) is(are) immobilized has the void defined by the structure containing the cover member and optionally the adhesive member, and the void may be a single space or 2 or more partitioned spaces. The 2 or more partitioned spaces may be provided, for example, by a partition structure as shown in Fig. 8. In the example shown in Fig. 8, the protruded portion 3A of the cover member and the substrate 1 are adhered to each other via the adhesive member 30A to provide the partitioned spaces 31. As another example wherein 2 or more partitioned spaces are provided, a partition structure as shown in Fig. 9 may also be provided. In the example shown in Fig. 9, the protruded portion 14 of the substrate and the cover member 3 are adhered to each other via the adhesive member 30B to provide the 2 or more partitioned spaces 31. Further, as another example, the 2 or more partitioned spaces may also be provided by partitioning the void only with the adhesive member 30A, with the protruded portion for providing the partition structure being provided neither in the substrate nor the cover member. In these examples wherein 2 or more partitioned spaces are provided, the spaces 31 are not communicating with each other, and each of these separately has the one or more penetrating holes 32 and liquid level-halting chambers 33. Like this, by providing 2 or more partitioned spaces, 2 or more kinds of the test substance solution may be applied to one analysis chip, so that 2 or more test substances may be assayed in one analysis chip at the same time.

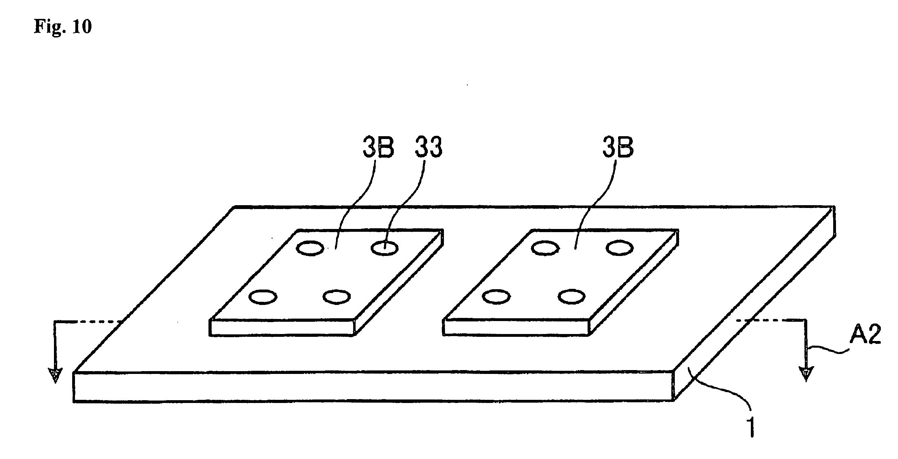

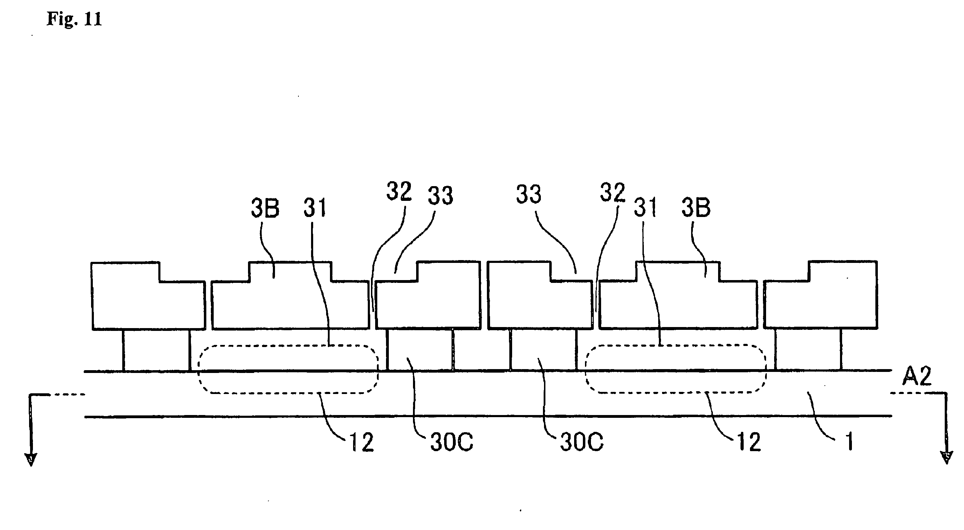

The analysis chip of the present invention may have a single cover member or may have 2 or more cover members per one substrate. Specifically, as shown in Fig. 10 or Fig. 11, one substrate 1 may have 2 or more cover members 3B. Each of the 2 or more cover members 3B may be provided on the substrate 1 through the separate adhesive members 30C. Preferably, each of the 2 or more cover members 3B may have the void 31 between the cover member and the substrate 1 and may have one or more penetrating holes communicating with each void, and each cover member 3B may separately have the region 12 in which the selective binding substance(s) is(are) immobilized. With such an embodiment, the cover member may be removed independently from each of the regions 12, so that independent usage may be carried out such that, for example, an analysis is carried out first with one of the regions 12, and the subsequent analysis is carried out with another region 12.

The material of the cover member constituting the analysis chip of the present invention is not restricted, and preferably a transparent material so that the condition of the solution is observable when the test substance solution is applied. Examples of such a material include glasses or plastics. Especially, from the view point of simplicity of preparation of structures such as penetrating holes and liquid level-halting chambers, a transparent resin such as polystyrene, polymethyl methacrylate, polycarbonate or the like may be preferably used. The method for preparation of the cover member is also not restricted, and it may be manufactured by cutting or injection molding. Injection molding is preferably used from the view point of availability in the mass production.

In the analysis chip of the present invention, the method by which the particles are injected (contained) in the substrate to which the cover member is attached is not restricted, and examples thereof include a method wherein an instrument having a tubular form in which the particles can pass through and having a thin tube which may be inserted into the penetrating hole of the cover member is used, which instrument is inserted into the penetrating hole of the cover member and the particles are made to pass through the instrument to be injected into the void. Examples of the instrument used herein may include pipettes, pipette tips, columns, capillaries and tubes. Alternatively, the particles may be added, before attachment of the cover member, to the region (the recessed portion 10 in Fig. 3, for example) of the substrate on which the selective binding substance(s) is(are) immobilized, and the cover member may be subsequently attached thereto.

A preferred example of the relationships among the irregular region, the cover member and the particles in the analysis chip of the present invention will now be explained referring to Fig. 12. In the example shown in Fig. 12, the selective binding substance(s) 45 such as DNA is(are) immobilized on the upper surfaces of the protruded portions 11 of the substrate 1. The particles (spherical beads, in this case) 2 are placed in the void of the recessed portion of the substrate 1. The selective binding substance(s) 45 and the particles 2 contact the solution containing the test substance (not shown). The test substance solution is retained in the void defined by the substrate 1, the adhesive member 30 and the cover member 3. In the example in Fig. 12, the shortest distance between the upper surface of the protruded portion of the substrate and the cover member 3 is less than the diameter of the particles 2. By this, the particles are not allowed to contact the upper surfaces of the protruded portions 11, so that damaging of the selective binding substance(s) 45 on the upper surface of the protruded portion 11 may be prevented. In cases where the particle is in a nonspherical shape such as an oval shape, the particles are similarly not allowed to contact the upper surface of the protruded portion 11 as long as the shortest distance between the upper surface of the protruded portion and the container is less than the smallest diameter of the particle, so that damaging of the selective binding substance(s) 45 may be prevented.

Such an analysis chip of the present invention may be used for analyses of various test substances. That is, a test substance is brought into contact with the substrate of the present invention on which a selective binding substance(s) is(are) immobilized, and the test substance is allowed to selectively bind to the selective binding substance(s), followed by assaying of presence/absence or the quantity of the test substance bound to the substrate via the selective binding substance(s), to analyze the test substance.

Examples of the test substance which may be subjected to the method for measurement using the analysis chip of the present invention include, but not limited to, nucleic acids to be measured such as genes of pathogenic bacteria, viruses and the like and causative genes of genetic diseases and the like, and parts thereof; various biological components having antigenecities; and antibodies to pathogenic bacteria, viruses and the like. Examples of the samples containing such test substances include, but not limited to, body fluids such as blood, serum, plasma, urine, feces, spinal fluid, saliva and various tissue fluids; various foods and beverages; and dilutions thereof. The nucleic acid which is used as a test substance may be one extracted from blood or cells by a conventional method and labeled, or may be one amplified by a nucleic acid-amplification method such as PCR using the nucleic acid as the template. In the latter case, the measurement sensitivity may be largely promoted. In cases where an amplification product of a nucleic acid is used as the test substance, the amplified nucleic acid can be labeled by carrying out the amplification in the presence of a nucleoside triphosphate labeled with a fluorescent substance or the like. In cases where the test substance is an antigen or an antibody, the antigen or antibody which is the test substance may be directly labeled by a conventional method. Alternatively, after binding the antigen or antibody which is the test substance with the selective binding substance(s), the substrate is washed, and a labeled antibody or antigen which undergoes antigen-antibody reaction is reacted with the antigen or antibody, followed by measurement of the amount of the label bound to the substrate.

In the method of the present invention for analysis of a test substance, the test substance is first brought into contact with the substrate constituting the analysis chip of the present invention, on which substrate a selective binding substance(s) is(are) immobilized, to allow selective binding between the test substance and the selective binding substance(s). That is, the test substance subjected to labeling, amplification or the like as described above is made to be an aqueous solution or dissolved in a buffer or the like to provide a solution (this may be referred to as "test substance solution" in the present specification), which is then brought into contact with the substrate.

Contacting of the test substance with the substrate on which the selective binding substance(s) is(are) immobilized may be carried out by injecting the test substance, which was made to be an aqueous solution or dissolved in an adequate buffer to provide a solution, into the irregular region on the substrate using a conventional instrument such as a pipette.

Before injecting the test substance solution into the analysis chip of the present invention to bring the solution into contact with the substrate on which the selective binding substance(s) is(are) immobilized, the solution is preferably subjected to a degassing treatment since this may effectively prevent generation of bubbles. Preferred examples of the method used for the degassing treatment include known methods such as a method wherein degassing is carried out using a vacuum pump or an aspirator to reduce pressure, a method wherein degassing is carried out by centrifugation, a method by ultrasonication, and a method by heating. Among these, a method wherein degassing is carried out using an aspirator or a vacuum pump to reduce pressure is more preferably used as an easy and simple method. The degree of vacuum in such cases may be one which does not cause bumping of the solution, and a pressure of 10 hPa (hectopascal) to 300 hPa, preferably 20 hPa to 200 hPa, more preferably 50 hPa to 100 hPa is used. The time for the degassing operation is preferably 2 minutes to 1 hour, more preferably 3 minutes to 30 minutes, still more preferably 5 minutes to 20 minutes.

When the analysis chip of the present invention is used, the test substance may be applied through the penetrating hole in the cover member, and the sealing member may be attached to the cover member to seal the penetrating hole, followed by selectively binding the test substance to the substrate constituting the analysis chip.

Application of the test substance through the penetrating hole may be carried out, for example, by injection through the penetrating hole with a conventional instrument such as a pipette.

Attaching of the sealing member to the cover member may be carried out in a manner wherein a part or all of, preferably all of, the penetrating holes are sealed. Preferred examples of the sealing member include flexible tapes such as adhesive tapes made of a polyimide film such as KAPTON (registered trademark of Du Pont-Toray Co., Ltd.) and adhesive tapes made of polyester, cellophane, vinyl chloride or the like, but the sealing member is not restricted thereto, and an arbitrary member which is non-flexible, plate-like and adhesive may be employed, or a shapeless sealing agent may be employed. From the view point of obtaining a better effect of the present invention by the liquid level-halting chamber, a flexible tape or a plate-like member is preferred and, from the view point of simplicity of operation and the like, a flexible tape is more preferred. In cases where a tape or a plate-like member is employed, the number of the member used is arbitrary. Specifically, all the penetrating holes on the cover member may be sealed with a single sealing member, or 2 or more sealing members may be employed, each of which members may be used to seal a part of the 2 or more sealing members. In cases where 2 or more cover members are provided on a single substrate as above, separate sealing members may be used for the individual cover members, or the penetrating holes on the 2 or more cover members may be sealed with a single sealing member at once. Usually, usage of one sealing member per one cover member is preferred since this may achieve simple and secure sealing.

Specific examples of sealing will now be explained referring to Fig. 12. In the example shown in Fig. 12, after application of the test substance solution (not shown) through the penetrating hole 32, a flexible adhesive tape 34 which is the sealing member is attached so as to cover the entire surface of the liquid level-halting chamber 33 to seal the penetrating holes. With such an embodiment, sealing, which is simple and does not cause leakage of the test substance solution and measurement errors, may be achieved.

In the analytical method of the present invention, selective binding means the process wherein the selective binding substance and the test substance are allowed to interact with each other to bind the test substance, via the selective binding substance, to the substrate on which the selective binding substance is immobilized. In the cases of the analysis chip of the present invention, since the particles move within the test substance solution by the weight, vibration and centrifugal force caused by moving and/or rotating of the chip, the selective binding may be allowed to proceed efficiently.

The reaction temperature and time for carrying out the selective binding are selected appropriately depending on the chain length of the nucleic acid of the test substance to be hybridized or the type(s) of the antigen and/or antibody involved in the immunoreaction, and in the cases of hybridization of nucleic acids, they are usually about 40°C to 70°C for 1 minutes to ten and several hours, and in the cases of immunoreaction, they are usually about room temperature to 50°C for 1 minute to several hours. The substrate on which the selective binding substance(s) is(are) immobilized may be moved and/or rotated as required to promote the selective binding.

The analysis chip of the present invention may efficiently stir the test substance solution by moving the particles during hybridization. Preferred examples of the method for moving the particles include a method wherein the analysis chip is rotated to make the particles fall to the direction of gravity; a method wherein the analysis chip containing the particles is placed on a shaker to shake or move the substrate; and a method wherein magnetic particles are used and the particles are moved by magnetic force. More preferably, a method wherein the chip is placed on a shaker and subjected to swirling rotation in a horizontal plane is used since, in such a case, the moving range of the particles is large and the particles move evenly, which results in an efficient stirring of the solution. In such a case, the number of revolution of the swirling rotation is preferably 10 to 1000 revolutions/minute, more preferably 100 to 500 revolutions/minute.

After finishing the selective binding, the chip may be usually subjected to the next step following removal of the cover member.

In the analytical method of the present invention, the above described selective binding is followed by measurement of the mass of the test substance bound to the substrate via the selective binding substance. This measurement may also be carried out in exactly the same manner as in the operation with the conventional analysis chip. For example, the mass of the test substance appropriately fluorescence-labeled and bound to the selective binding substance may be measured by reading of its fluorescence intensity with a known scanner or the like.

In the analytical method of the present invention, in cases where a nucleic acid is immobilized as the selective binding substance, a nucleic acid having a sequence complementary to this nucleic acid or to a part thereof may be measured. In cases where an antibody or an antigen was immobilized as the selective binding substance, an antigen or antibody which immunologically reacts with this antibody or antigen may be measured. As used herein, "measurement" includes both detection and quantification.

### EXAMPLES

The present invention will now be explained in more detail by way of Examples below. The present invention is not restricted to the Examples below.

### Example 1

### (1) Preparation of Substrate for Analysis Chip

Using the LIGA (Lithographie Galvanoformung Abformung) process which is a known method, a mold for injection molding was prepared, and a substrate made of polymethyl methacrylate (PMMA) having a shape as described later was obtained by injection molding. The average molecular weight of the PMMA used was 50,000, and carbon black (#3050B produced by Mitsubishi Chemical) was included therein at a proportion of 1% by weight to make the substrate black. Results of measurement of the spectral reflectance and spectral transmittance of this black substrate showed not more than 5% of the spectral reflectance at any wavelength within the visible wavelength region (400 nm to 800 nm) and not less than 0.5% of transmittance within the same range of the wavelength. Both the spectral reflectance and the spectral transmittance did not show a specific spectral pattern (such as a peak) within the visible wavelength region, and the spectrum was evenly flat. The spectral reflectance was measured with specular reflection from the substrate using a device (CM-2002 produced by Minolta Camera) having an illumination/light-receiving optical system satisfying the condition C of JIS Z 8722.

The substrate used (hereinafter referred to as "substrate A") had the shape exemplified in Fig. 3 and Fig. 4 and external dimensions of a longitudinal length of 76 mm, a lateral length of 26 mm and a thickness of 1 mm. At the center of the substrate, a recessed portion (corresponding to the recessed portion 10 in Fig. 3) having dimensions of a longitudinal length of 39.4 mm, a lateral length of 19.0 mm and a depth of 0.15 mm was provided, in which recessed portion 9248 protruded portions (corresponding to the protruded portions 11 in Fig. 3) having a diameter of 0.1 mm and a height of 0.15 mm were provided. In this substrate A, the difference in height between the upper surfaces of the protruded portions (corresponding to the protruded portions 11 in Fig. 3) and the upper surface of the flat area (corresponding to the protruded portion 13) (the average height of the protruded portions) was not more than 3 µm. Variation in height of the upper surface of the protruded portion (corresponding to the protruded portion 13) (difference between the height of the highest part of the upper surface of the protruded portion and the height of the lowest part of the upper surface of the protruded portion) was not more than 3 µm. The pitch between the protruded portions (L1 in Fig. 4; distance between the center of a protruded portion and the center of an adjacent protruded portion) was 0.5 mm.

The above substrate A was immersed in aqueous 10N sodium hydroxide solution at 70°C for 12 hours. This was washed sequentially with pure water, 0.1N HCl solution, and pure water, and carboxyl groups were formed on the surface of the substrate.

### (2) Immobilization of Selective Binding Substances

Each of oligonucleotides was immobilized on the substrate A as the selective binding substances (probe DNAs) under the following condition. The DNA microarray oligonucleotide set "Homo sapiens (human) AROS V4.0 (60 bases each)" produced by Operon Biotechnologies was used as the oligonucleotides. These oligonucleotides were dissolved in pure water to a final concentration of 0.3 nmol/µL and used as stock solutions. When the stock solution was spotted on the substrate, it was 10-fold diluted with PBS (prepared by combining 8 g of NaCl, 2.9 g of Na₂HPO₄·12H₂O, 0.2 g of KCl and 0.2 g of KH₂PO₄, dissolving thereof in pure water to attain a final volume of 1 L, and then adjusting pH of the resulting solution to 5.5 by addition of hydrochloric acid) to attain a final concentration of 0.03 nmol/µL for the probe DNA, in which solution 1-ethyl-3-(3-dimethylaminopropyl)carbodiimide (EDC) was added to a final concentration of 50 mg/mL to allow condensation between the carboxyl group formed on the surface of the substrate made of PMMA and the terminal amino group of the probe DNA. The solutions were respectively spotted on the upper surfaces of all the protruded portions of the substrate A using an arrayer (spotter) (Gene Stamp-II produced by Nippon Laser & Electronics Lab.). Subsequently, the spotted substrate was incubated in a plastic container at 37°C and a humidity of 100% for about 20 hours. Finally, the substrate was washed with pure water and dried by centrifugation using a spin drier.

### (3) Attachment of Cover Member to Analysis Chip Substrate

The cover member was attached as follows to the substrate A on which the selective binding substances were immobilized.

A PMMA flat plate with dimensions of a longitudinal length of 41.4 mm, a lateral length of 21 mm and a thickness of 1 mm was prepared by cutting and used as the cover member. The penetrating holes and the liquid level-halting chambers were provided in the prepared cover member as exemplified in 32 and 33 in Fig. 7. A double-stick tape with a width of 1 mm was used as the adhesive member, and was attached along the longitudinal fringe of 41.4 mm and the lateral fringe of 21 mm such that the tape was laminated at a thickness of 50 µm, to attach the cover member to the substrate A.

### (4) Surfactant-coating of Surface of Particles for Stirring

In a stainless steel vat (10cm x 10cm x 5cm), 10 g of particles made of zirconia having a diameter of 180 µm (produced by Toray Industries, Inc.) were placed, and 50 mL of aqueous 0.1% sodium dodecyl sulfate (SDS) solution was added thereto as the surfactant. After sonication for 10 minutes, the supernatant (SDS component) was removed, and the particles were dried at 70°C for 12 hours using an oven. The surface roughness of the particle before the treatment was 165 nm in terms of the centerline average roughness (Ra) of its surface. The measurement of the Ra value of the surface of the particle was carried out with an electron scanning microscope (ESA-2000 produced by Elionix Co., Ltd.) after vacuum deposition of Au on its surface. The centerline average roughness was measured for arbitrarily selected 10 particles at a magnification of x10,000 and with a cut off value of 0, and the average value was calculated.

### (5) Injection (Containing) of Particles in Analysis Chip and Evaluation of Operability of Injection (Containing)

In the substrate A to which the cover member was attached in the above (3), 120 mg of the particles made of zirconia coated with the surfactant in the above (4) was injected (contained) in the void formed by the substrate A and the cover member (the recessed portion of the irregular region of the surface of the substrate A). Injection of the particles was carried out through the penetrating hole of the cover member (the penetrating hole 32 exemplified in Fig. 7 or Fig. 12). The thus obtained analysis chip is hereinafter referred to as "analysis chip 1".

Here, operability of injection of the particles into the analysis chip was evaluated as follows. When 120 mg of the particles were injected, the operability was evaluated as "A" in cases where the time required for the injection was less than 3 minutes since the operation was very easy; it was evaluated as "B" in cases where the time required for the injection was not less than 3 minutes and less than 5 minutes since the operation was relatively easy; and it was evaluated as "C" in cases where the injection of the whole quantity of the particles was not accomplished within 5 minutes since the operation was difficult or not easy.

The operability of injection of the particles in the analysis chip 1 was "A" (Table 1).

### (6) Preparation of Test Substance DNA

As the test substance, aRNA (antisense RNA) which is common as a test substance was used. From 5µg of the total RNA (Human Reference RNA produced by CLONTECH) which is commercially available and was derived from human cultured cells, 5µg of Cy3-labeled aRNA was obtained using an aRNA preparation kit produced by Ambion.

In the present Example, the following Examples and the Comparative Examples, the test substance solution used for hybridization was one prepared by diluting the above prepared labeled aRNA with a solution of 1 wt% BSA, 5 x SSC, 0.01 wt% salmon sperm DNA and 0.1 wt% SDS (each concentration represents a final concentration) unless otherwise specified.

### (7) Hybridization Reaction and Evaluation of Number of Generated Bubbles

Using a micropipette, 165 µL of the hybridization test substance solution containing 200 ng of Cy3-labeled aRNA was injected through the penetrating hole into the void (reaction vessel) between the substrate A and the cover member of the analysis chip 1. The solution could be easily injected at this time, and no bubble was entrapped. Using the KAPTON tape (As One Corporation) as a sealing material, 4 penetrating holes were sealed. A hybridization chamber (Takara Hybridization chamber (produced by Takara Bio Inc.)) was closely contacted with and fixed to a sheet shaking platform (MMS FIT-S produced by Tokyo Rikakikai), and the analysis chip 1 was placed in the hybridization chamber. At this time, 15 µL each of ultrapure water was dropped to recesses at the both sides of the location where the analysis chip 1 was placed. After closing the lid of the hybridization chamber, the chip was fixed by tightening of 6 fixation screws, and the chamber was fixed on a shaker (MMS-310 produced by Tokyo Rikakikai) installed in an thermostat chamber (FMS-1000 produced by Tokyo Rikakikai) set at 42°C. The front side of the thermostat chamber was shaded with aluminum foil, and the chamber was incubated with rotary shaking at 250 revolutions/minute at 42°C for 16 hours. After the incubation, the analysis chip 1 was removed from the hybridization chamber.

Through the cover member, bubbles in the test substance solution observed on the substrate A of the analysis chip 1 were counted. Based on the results obtained by 10 runs of hybridization reaction using the analysis chip 1, the number of the bubbles generated in the test substance solution was 4.5 on average per reaction (Table 1).

### (8) Measurement of Fluorescence Signal and Evaluation of Deviation of Detection Sensitivity

After removal of the cover member and the double-stick tape adhered to the substrate A of the analysis chip 1, the substrate A was washed and dried. The substrate A after the above treatment was placed in a scanner for a DNA chip (GenePix 4000B produced by Axon Instruments), and the signal value of the label (fluorescence intensity) of the test substance subjected to the hybridization reaction and the background noise were measured under a condition wherein the laser output was 33% and the voltage setting for the photomultiplier was 500. Among the totally 9248 spots, 32 spots were used as negative control spots for measurement of the background fluorescence, and the true signal value for each spot was calculated by subtracting the background signal value from individual signal values.

For evaluation of deviation of the detection sensitivity due to unevenness of the hybridization reaction, 10 runs of hybridization reaction was carried out using the analysis chip 1, and the deviation of the background signal values (CV value = the standard deviation of the background signal values in all runs / the mean value of the background signal values of all runs (%)) was calculated for each reaction. As a result, the average of the deviations (CV values) of the background signal values based on the 10 runs of evaluation was 8.4% (Table 1).

### Example 2

Evaluation using the analysis chip 1 was carried out in the same manner as in Example 1 except that the test substance solution prepared in Example 1 (6) was subjected to a degassing treatment as follows.

To a 0.2 mL PCR tube (72.737.002 produced by ASSIST), 175 µL of the test substance solution was added, and the tube was placed in a degasifier (type NDA-015 aspirator produced by ULVAC) with the lid left open to carry out degassing of the solution. The ultimate pressure during degassing was 50 hPa according to indication by the apparatus, and the time period for degassing was 25 minutes.

In the same manner as in Example 1 (7), bubbles in the test substance solution observed on the substrate after the hybridization reaction were counted. The average number of the bubbles per reaction calculated from the results obtained by 6 runs of the reaction was 0.4 (Table 1).

Further, in the same manner as in Example 1 (8), the deviation of the background signal values (CV value) was calculated, and shown to be 6.7% (Table 1).

### Comparative Example 1

The "analysis chip 2" was prepared in the same manner as in Example 1 except that the particles made of zirconia were used as they were without being subjected to coating with a surfactant (Example 1 (4)). Using this analysis chip 2, evaluation was carried out as in Example 1.

The result of evaluation of injection operability of the particles as in Example 1 (5) showed that inclusion of the particles into the analysis chip 2 was difficult and the time required exceeded 5 minutes, so that the operability was evaluated as "C" (Table 1).

For this analysis chip 2, bubbles generated in the test substance solution after the hybridization reaction were counted in the same manner as in Example 1 (7). The average number of the bubbles calculated from the results obtained by 24 runs of the reaction was 13.0 (Table 1).

Further, in the same manner as in Example 1 (8), the deviation of the background signal values (CV value) was calculated, and shown to be 12.1% (Table 1).

### Comparative Example 2

Evaluation was carried out in the same manner as in Example 2 except that the analysis chip 2 prepared in Comparative Example 1 was used instead of the analysis chip 1, with a degassing treatment of the test substance solution.

Bubbles generated after the hybridization reaction were counted in the same manner as in Example 1 (7). The average number obtained by 3 runs of evaluation was 9.0 (Table 1).

Further, in the same manner as in Example 1 (8), the deviation of the background signal values (CV value) was calculated, and shown to be 10.5% (Table 1).

### Example 3

In Example 1 (4), as the surfactant, sodium deoxycholate (a kind of anionic surfactant) was used instead of sodium dodecyl sulfate (SDS), and 120 mg of the particles subjected to a coating treatment with the surfactant in the same manner was injected to prepare an "analysis chip 3". Using this analysis chip 3, evaluation was carried out with a degassing treatment of the test substance solution as in Example 2.

The result of evaluation of injection operability of the particles as in Example 1 (5) showed that inclusion operability of the particles into the analysis chip 3 was evaluated as "B" since the average of the runs required in 10 runs of the reaction was not less than 3 minutes and not more than 5 minutes (Table 2).

For this analysis chip 3, bubbles generated in the test substance solution after the hybridization reaction were counted in the same manner as in Example 1 (7). The average number of the bubbles calculated from the results obtained by 10 runs of the reaction was 0.6 (Table 2).

Further, in the same manner as in Example 1 (8), the deviation of the background signal values (CV value) was calculated, and shown to be 7.2% (Table 1).

### Example 4

In Example 1 (4), as the surfactant, Pluronic F68 (a kind of nonionic surfactant) was used instead of sodium dodecyl sulfate (SDS), and the particles subjected to a coating treatment with the surfactant were used by the following steps to prepare an "analysis chip 4". That is, after treating the particles made of zirconia in the same manner as in Example 1 (4), the particles were washed once with 400 mL of deionized water (Milli-Q water) and then dried at 70°C for 4 hours. To the analysis chip, 120 mg of these particles were injected to prepare the analysis chip 4.

By all the results obtained by 10 runs of evaluation of injection operability of the particles in the same manner as in Example 1 (5), the time required was shown to be not more than 3 minutes. Thus, the operability was evaluated as "A" (Table 2).

To the analysis chip 4, 165 µL of the test substance solution subjected to a degassing treatment in the same manner as in Example 2 was applied, and hybridization reactions were carried out in the same manner as in Example 1 (7). The number of bubbles generated in the test substance solution was counted, and the average number obtained by 6 runs of evaluation was 1.2 (Table 2).

Further, in the same manner as in Example 1 (8), the deviation of the background signal values (CV value) was calculated, and shown to be 8.4% (Table 2).

### Example 5

In Example 1 (4), as the surfactant, Pluronic F127 (a kind of nonionic surfactant) was used instead of sodium dodecyl sulfate (SDS), and the particles subjected to a coating treatment with the surfactant were used by the following steps to prepare an "analysis chip 5". That is, after treating the particles made of zirconia in the same manner as in Example 1 (4), the particles were washed once with 400 mL of deionized water (Milli-Q water) and then dried at 70°C for 4 hours. To the analysis chip, 120 mg of these particles were injected to prepare the analysis chip 5.

By all the results obtained by 10 runs of evaluation of injection operability of the particles in the same manner as in Example 1 (5), the time required was shown to be not more than 3 minutes. Thus, the operability was evaluated as "A" (Table 2).

To the analysis chip 5, 165 µL of the test substance solution subjected to a degassing treatment in the same manner as in Example 2 was applied, and hybridization reactions were carried out in the same manner as in Example 1 (7). The number of bubbles generated in the test substance solution was counted, and the average number obtained by 6 runs of evaluation was 1.8 (Table 2).

Further, in the same manner as in Example 1 (8), the deviation of the background signal values (CV value) was calculated, and shown to be 7.6% (Table 2).

### Example 6

In Example 1 (4), as the surfactant, Triton X-100 (a kind of nonionic surfactant) was used instead of sodium dodecyl sulfate (SDS), and the particles subjected to a coating treatment with the surfactant were used by the following steps to prepare an "analysis chip 6". That is, after treating the particles made of zirconia in the same manner as in Example 1 (4), the particles were subjected to ultrasonic washing in 400 mL of deionized water (Milli-Q water) for 30 seconds and then dried at 70°C for 4 hours. To the analysis chip, 120 mg of these particles were injected to prepare the analysis chip 6.

By the results obtained by 10 runs of evaluation of injection operability of the particles in the same manner as in Example 1 (5), the time required was shown to be not more than 3 minutes in 7 chips, and not less than 3 minutes and not more than 5 minutes in 3 chips. Thus, the operability was evaluated as "B" (Table 2).

To the analysis chip 6, 165 µL of the test substance solution subjected to a degassing treatment in the same manner as in Example 2 was applied, and hybridization reactions were carried out in the same manner as in Example 1 (7). The number of bubbles generated in the test substance solution was counted, and the average number obtained by 6 runs of evaluation was 2.1 (Table 2).

Further, in the same manner as in Example 1 (8), the deviation of the background signal values (CV value) was calculated, and shown to be 8.3% (Table 2).

### Example 7

In Example 1 (4), Tween 20 (a kind of nonionic surfactant) was used instead of sodium dodecyl sulfate (SDS) as the surfactant, and the particles subjected to a coating treatment with the surfactant were used by the following steps to prepare an "analysis chip 7". That is, after treating the particles made of zirconia in the same manner as in Example 1 (4), the particles were washed once with 400 mL of deionized water (Milli-Q water) and then dried at 70°C for 4 hours. To the analysis chip, 120 mg of these particles were injected to prepare the analysis chip 7.

By the results obtained by 10 runs of evaluation of injection operability of the particles in the same manner as in Example 1 (5), the time required was shown to be not more than 3 minutes for 6 chips, and not less than 3 minutes and not more than 5 minutes for 4 chips. Thus, the operability was evaluated as "B" (Table 2).

To the analysis chip 7, 165 µL of the test substance solution subjected to a degassing treatment in the same manner as in Example 2 was applied, and hybridization reactions were carried out in the same manner as in Example 1 (7). The number of bubbles generated in the test substance solution was counted, and the average number obtained by 6 runs of evaluation was 2.2 (Table 2).

Further, in the same manner as in Example 1 (8), the deviation of the background signal values (CV value) was calculated, and shown to be 7.0% (Table 2).

### Example 8

In Example 1 (4), as the surfactant, 3-[(3-cholamidopropyl)dimethylammonio]-2-hydroxypropanesulfonate (CHAPSO; a kind of amphoteric surfactant) was used instead of sodium dodecyl sulfate (SDS), and the particles subjected to a coating treatment with the surfactant were used by the following steps to prepare an "analysis chip 8". That is, after treating the particles made of zirconia in the same manner as in Example 1 (4), the particles were washed once with 400 mL of deionized water (Milli-Q water) and then dried at 70°C for 4 hours. To the analysis chip, 120 mg of these particles were injected to prepare the analysis chip 8.

By the results obtained by 10 runs of evaluation of injection operability of the particles in the same manner as in Example 1 (5), the time required was shown to be not more than 3 minutes for 8 chips, and not less than 3 minutes and not more than 5 minutes for 2 chips. Thus, the operability was evaluated as "B" (Table 2).

To the analysis chip 8, 165 µL of the test substance solution subjected to a degassing treatment in the same manner as in Example 2 was applied, and hybridization reactions were carried out in the same manner as in Example 1 (7). The number of bubbles generated in the test substance solution was counted, and the average number obtained by 6 runs of evaluation was 2.5 (Table 2).

Further, in the same manner as in Example 1 (8), the deviation of the background signal values (CV value) was calculated, and shown to be 7.6% (Table 2).

### Example 9

In Example 1 (4), as the surfactant, cetyltrimethylammonium bromide (CTAB; a kind of cationic surfactant) was used instead of sodium dodecyl sulfate (SDS), and the particles subjected to a coating treatment with the surfactant were used by the following steps to prepare an "analysis chip 9". That is, after treating the particles made of zirconia in the same manner as in Example 1 (4), the particles were washed once with 400 mL of deionized water (Milli-Q water) and then dried at 70°C for 4 hours. To the analysis chip, 120 mg of these particles were injected to prepare the analysis chip 9.

By the results obtained by 10 runs of evaluation of injection operability of the particles in the same manner as in Example 1 (5), the time required was shown to be not more than 3 minutes for 7 chips, and not less than 3 minutes and not more than 5 minutes for 3 chips. Thus, the operability was evaluated as "B" (Table 2).

To the analysis chip 9, 165 µL of the test substance solution subjected to a degassing treatment in the same manner as in Example 2 was applied, and hybridization reactions were carried out in the same manner as in Example 1 (7). The number of bubbles generated in the test substance solution was counted, and the average number obtained by 6 runs of evaluation was 3.0 (Table 2).

Further, in the same manner as in Example 1 (8), the deviation of the background signal values (CV value) was calculated, and shown to be 7.9% (Table 2).

### Example 10

In Example 1 (1), the "substrate B" which is a substrate having only a recessed portion with dimensions of a longitudinal length of 39.4 mm, a lateral length of 19.0 mm and a depth of 0.05 mm (one having the same shape as exemplified in Fig. 3 and Fig. 4 except that it does not have the protruded portions 11) was used as the substrate of the analysis chip instead of the substrate A. Immobilization of the selective binding substances in Example 1 (2) was carried out by placing 9248 spots with the same intervals as those for the substrate A so as to form a rectangle with dimensions of a longitudinal length of 39.4 mm and a lateral length of 19.0 mm on the bottom surface of the recessed portion.

As the cover member of Example 1 (3), a PMMA flat plate with dimensions of a longitudinal length of 41.4 mm, a lateral length of 21 mm and a thickness of 1 mm was prepared by cutting and used. The penetrating holes and the liquid level-halting chambers were provided in the prepared cover member as exemplified as 32 and 33 in Fig. 7. A double-stick tape with a width of 1 mm was used as the adhesive member, and was attached along the longitudinal fringe of 41.4 mm and the lateral fringe of 21 mm such that the tape was laminated at a thickness of 50 µm, to attach the cover member to the substrate B.

Except these, by the same steps as those in (1)-(4) in Example 1, the "analysis chip 10" into which 120 mg of the particles made of zirconia coated with sodium dodecyl sulfate (SDS) as the surfactant were injected was prepared.

By all the results obtained by 10 runs of evaluation of injection operability of the particles in the same manner as in Example 1 (5), the time required was shown to be not less than 3 minutes and not more than 5 minutes. Thus, the operability was evaluated as "B" (Table 3).

To the analysis chip 10, 165 µL of the test substance solution subjected to a degassing treatment in the same manner as in Example 2 was applied, and hybridization reactions were carried out in the same manner as in Example 1 (7). The number of bubbles generated in the test substance solution was counted, and the average number obtained by 6 runs of evaluation was 0.5 (Table 3).

Further, in the same manner as in Example 1 (8), the deviation of the background signal values (CV value) was calculated, and shown to be 9.5% (Table 3).

### Example 11

In Example 1 (1), the "substrate C" which is a flat plate having neither a recessed portion nor a protruded portion (one having the same shape as exemplified in Fig. 3 and Fig. 4 except that it has neither the recessed portion 10 nor the protruded portion 11) was used as the substrate of the analysis chip instead of the substrate A. Immobilization of the selective binding substances in Example 1 (2) was carried out by placing 9248 spots with the same intervals as those for the substrate A so as to form a rectangle with dimensions of a longitudinal length of 39.4 mm and a lateral length of 19.0 mm on the upper flat surface of the substrate C.

As the cover member of Example 1 (3), a PMMA flat plate with dimensions of a longitudinal length of 41.4 mm, a lateral length of 21 mm and a thickness of 1 mm was prepared by cutting and used. The penetrating holes and the liquid level-halting chambers were provided in the prepared cover member as exemplified in 32 and 33 in Fig. 7. A double-stick tape with a width of 1 mm was used as the adhesive member, and was attached along the longitudinal fringe of 41.4 mm and the lateral fringe of 21 mm such that the tape was laminated at a thickness of 200 µm, to attach the cover member to the substrate C.

Except these, by the same steps as those in (1)-(4) in Example 1, the "analysis chip 11" in which 120 mg of the particles made of zirconia coated with sodium dodecyl sulfate (SDS) as the surfactant were injected was prepared.

By the results obtained by 10 runs of evaluation of injection operability of the particles in the same manner as in Example 1 (5), the time required was shown to be not more than 3 minutes for 7 chips, and not less than 3 minutes and not more than 5 minutes for 3 chips. Thus, the operability was evaluated as "B" (Table 3).

To the analysis chip 11, 165 µL of the test substance solution subjected to a degassing treatment in the same manner as in Example 2 was applied, and hybridization reactions were carried out in the same manner as in Example 1 (7). The number of bubbles generated in the test substance solution was counted, and the average number obtained by 6 runs of evaluation was 0.6 (Table 3).

Further, in the same manner as in Example 1 (8), the deviation of the background signal values (CV value) was calculated, and shown to be 9.3% (Table 3).

### Example 12

In Example 1 (4), particles made of iron with a diameter of 200 µm (produced by Sanshokenmazai Co., Ltd.) were used as the particles instead of the particles made of zirconia with a diameter of 180 µm, and 120 mg of the particles subjected to a coating treatment with sodium dodecyl sulfate (SDS) as the surfactant in the same manner as in Example 1 (4) were injected to prepare an "analysis chip 12".

By all the results obtained by 10 runs of evaluation of injection operability of the particles in the same manner as in Example 1 (5), the time required was shown to be not less than 3 minutes and not more than 5 minutes. Thus, the operability was evaluated as "B" (Table 3).

The test substance solution subjected to a degassing treatment in the same manner as in Example 2 was applied to the analysis chip 12, and hybridization reactions were carried out in the same manner as in Example 1 (7). The number of bubbles generated in the test substance solution was counted, and the average number obtained by 6 runs of evaluation was 2.2 (Table 3).

Further, in the same manner as in Example 1 (8), the deviation of the background signal values (CV value) was calculated, and shown to be 8.0% (Table 3).

### Comparative Example 3

A chip was prepared in the same manner as in Example 12 except that the particles made of iron with a diameter of 200 µm were used as they were as the particles, to prepare an "analysis chip 13". Using this analysis chip 13, evaluation was carried out in the same manner as in Example 1.

By all the results obtained by 10 runs of evaluation of injection operability of the particles in the same manner as in Example 1 (5), the injection operation was shown to be difficult and take more than 5 minutes. Thus, the operability was evaluated as "C" (Table 3).

The test substance solution subjected to a degassing treatment in the same manner as in Example 2 was applied to the analysis chip 13, and hybridization reactions were carried out in the same manner as in Example 1 (7). The number of bubbles generated in the test substance solution was counted, and the average number obtained by 6 runs of evaluation was 10.0 (Table 3).

Further, in the same manner as in Example 1 (8), the deviation of the background signal values (CV value) was calculated, and shown to be 13.3% (Table 3).

### Example 13

In Example 1 (4), particles made of glass with a diameter of 200 µm (produced by Bio Medical Science Inc.) were used as the particles instead of the particles made of zirconia with a diameter of 180 µm, and 120 mg of the particles subjected to a coating treatment with sodium dodecyl sulfate (SDS) as the surfactant in the same manner as in Example 1 (4) were injected to prepare an "analysis chip 14".

By all the results obtained by 10 runs of evaluation of injection operability of the particles in the same manner as in Example 1 (5), the time required was shown to be not less than 3 minutes and not more than 5 minutes. Thus, the operability was evaluated as "B" (Table 3).

The test substance solution subjected to a degassing treatment in the same manner as in Example 2 was applied to the analysis chip 14, and hybridization reactions were carried out in the same manner as in Example 1 (7). The number of bubbles generated in the test substance solution was counted, and the average number obtained by 6 runs of evaluation was 1.0 (Table 3).

Further, in the same manner as in Example 1 (8), the deviation of the background signal values (CV value) was calculated, and shown to be 8.2% (Table 3).

### Comparative Example 4

A chip was prepared in the same manner as in Example 13 except that, as the particles, those made of glass with a diameter of 200 µm were used as they were without the coating treatment with the surfactant (Example 1 (4)), to prepare an "analysis chip 15". Using this analysis chip 15, evaluation was carried out in the same manner as in Example 1.

By all the results obtained by 10 runs of evaluation of injection operability of the particles in the same manner as in Example 1 (5), the injection operation was shown to be difficult and take more than 5 minutes. Thus, the operability was evaluated as "C" (Table 3).

The test substance solution subjected to a degassing treatment in the same manner as in Example 2 was applied to the analysis chip 15, and hybridization reactions were carried out in the same manner as in Example 1 (7). The number of bubbles generated in the test substance solution was counted, and the average number obtained by 6 runs of evaluation was 8.8 (Table 3).

Further, in the same manner as in Example 1 (5), the deviation of the background signal values (CV value) was calculated to be 12.9% (Table 3).

**[Table 1]**

| | Example 1 | Example 2 | Comparative Example 1 | Comparative Example 2 |
|---|---|---|---|---|
| Analysis chip number Substrate | 1 Substrate A | 1 Substrate A | 2 Substrate A | 2 Substrate A |
| Treatment with surfactant | Yes | Yes | No | No |
| Surfactant | SDS | SDS | - | - |
| Degassing treatment | No | Yes | No | Yes |
| Material of particles | Zirconia | Zirconia | Zirconia | Zirconia |
| Injection operability of particles | A | A | C | C |
| Average number of bubbles generated | 4.5 | 0.4 | 13.0 | 9.0 |
| CV value (%) of background signals | 8.4 | 6.7 | 12.1 | 10.5 |

**[Table 2]**

| | Example 3 | Example 4 | Example 5 | Example 6 | Example 7 | Example 8 | Example 9 |
|---|---|---|---|---|---|---|---|
| Analysis chip number Substrate | 3 Substrate A | 4 Substrate A | 5 Substrate A | 6 Substrate A | 7 Substrate A | 8 Substrate A | 9 Substrate A |
| Treatment with surfactant | Yes | Yes | Yes | Yes | Yes | Yes | Yes |
| Surfactant | Sodium deoxycholate | Pluronic F68 | Pluronic F127 | Triton X-100 | Tween 20 | CHAPSO | CTAB |
| Degassing treatment | Yes | Yes | Yes | Yes | Yes | Yes | Yes |
| Marterials of particles | Zirconia | Zirconia | Zirconia | zirconia | zirconia | Zirconia | Zirconia |
| Injection operability of particles | B | A | A | B | B | B | B |
| Average number of bubbles generated | 0.6 | 1.2 | 1.8 | 2.1 | 2.2 | 2.5 | 3.0 |
| CV value (%) of background signals | 7.2 | 8.4 | 7.6 | 8.3 | 7.0 | 7.6 | 7.9 |

**[Table 3]**

| | Example 10 | Example 11 | Example 12 | Comparative Example 3 | Example 13 | Comparative Example 4 |
|---|---|---|---|---|---|---|
| Analysis chip number Substrate | 10 Substrate B | 11 Substrate C | 12 Substrate A | 13 Substrate A | 14 Substrate A | 15 Substrate A |
| Treatment with surfactant | Yes | Yes | Yes | No | Yes | No |
| Surfactant | SDS | SDS | SDS | - | SDS | - |
| Degassing treatment | Yes | Yes | Yes | Yes | Yes | Yes |
| Material of particles | Zirconia | Zirconia | Iron | Iron | Glass | Glass |
| Injection operability of particles | B | B | B | C | B | C |
| Average number of bubbles generated | 0.5 | 0.6 | 2.2 | 10.0 | 1.0 | 8.8 |
| CV value (%) of background signals | 9.5 | 9.3 | 8.0 | 13.3 | 8.2 | 12.9 |

From the results in the above Examples 1-13 and Comparative Examples 1-4, it was revealed that generation of bubbles is suppressed by coating the surface of the particles with a surfactant, so that deviation of data (CV value of the background signals) may be reduced and operability of injection of the particles into the analysis chip may be improved, and that the generation of the bubbles may be more effectively suppressed when a degassing treatment of the test substance solution is carried out in combination with this coating.

### INDUSTRIAL APPLICABILITY

The present invention suppresses the deviation of detection sensitivities and enables detection of a test substance with high sensitivity in an analysis chip having a substrate on which a selective binding substance(s) capable of selectively binding to the test substance is(are) immobilized, wherein the test substance solution may be stirred with particles. The analysis chip provided by the present invention is useful as an analysis chip for detection of various biologically relevant substances in the fields of medicine and healthcare, and also as an analysis chip for detection of trace substances in the fields of food and environment.

## Claims

1. An analysis chip comprising:
a substrate having a surface on which a selective binding substance(s) is (are) immobilized;
a cover member adhered to said substrate;
a void between said substrate and said cover member; and
particles movably contained or injected in said void;
the surfaces of said particles being coated with a surfactant(s).

2. The analysis chip according to claim 1, wherein said surfactant coated on said surfaces of said particles is an anionic surfactant or a nonionic surfactant.

3. The analysis chip according to claim 1 or 2, wherein said substrate comprises an irregular region composed of recessed portions and protruded portions, and said selective binding substance(s) is(are) immobilized on upper surfaces of said protruded portions.

4. The analysis chip according to any one of claims 1 to 3, wherein the material constituting said particles coated with said surfactant(s) comprises a ceramic.

5. The analysis chip according to any one of claims 1 to 4, wherein one or more penetrating holes communicating with said void are formed in said cover member.

6. The analysis chip according to any one of claims 1 to 5, wherein the shortest distance between said surface of said substrate, on which said selective binding substance(s) is(are) immobilized, and said cover member is smaller than the diameter of said particles.

7. The analysis chip according to any one of claims 1 to 6, wherein said selective binding substance is a DNA, RNA, protein, peptide, saccharide, sugar chain or lipid.

8. A method for analyzing a test substance, said method comprising the steps of:
bringing said analysis chip according to any one of claims 1 to 6 into contact with a solution containing a test substance, thereby selectively binding said test substance to said selective binding substance immobilized on the surface of said substrate; and
measuring the amount of said substance bound to said analysis chip through said selective binding substance.

9. The method for analyzing a test substance, according to claim 8, wherein said solution containing said test substance is subjected to a degassing treatment before bringing said solution containing said test substance into contact with said analysis chip.

## Patentansprüche

1. Analysechip, der folgendes aufweist:
einen Träger mit einer Oberfläche, auf der eine (mehrere) selektiv bindende(n) Substanz(en) immobilisiert ist (sind);
ein Abdeckteil, das am Träger klebt;
einen Hohlraum zwischen dem Träger und dem Abdeckteil; und
Partikel, die beweglich in den Hohlraum enthalten oder in diesen eingespritzt sind;
wobei die Oberfläche der Partikel mit einem (mehreren) oberflächenaktiven Mittel(n) überzogen ist.

2. Analysechip nach Anspruch 1, wobei das auf die Oberfläche der Partikel aufgetragene oberflächenaktive Mittel ein anionisches oder ein nichtionisches oberflächenaktives Mittel ist.

3. Analysechip nach Anspruch 1 oder 2, wobei der Träger einen unregelmäßigen Bereich aufweist, der aus vertieften Abschnitten und vorstehenden Abschnitten aufgebaut ist, und wobei die selektive bindende(n) Substanz(en) auf der Oberseite der vorstehenden Abschnitte immobilisiert ist (sind).

4. Analysechip nach einem der Ansprüche 1 bis 3, wobei das Material, das die Partikel bildet, die mit dem (den) oberflächenaktiven Mittel(n) überzogen sind, eine Keramik umfaßt.

5. Analysechip nach einem der Ansprüche 1 bis 4, wobei ein oder mehrere durchdringende Löcher, die mit dem Hohlraum in Verbindung stehen, in dem Abdeckteil ausgebildet sind.

6. Analysechip nach einem der Ansprüche 1 bis 5, wobei der geringste Abstand zwischen der Oberfläche des Trägers, auf dem die selektiv bindende(n) Substanz(en) immobilisiert ist (sind), und dem Abdeckteil kleiner als der Durchmesser der Partikel ist.

7. Analysechip nach einem der Ansprüche 1 bis 6, wobei die selektiv bindende Substanz eine DNA, RNA, ein Protein, Peptid, Saccharid, eine Zuckerkette oder ein Lipid ist.

8. Verfahren zum Analysieren einer Testsubstanz, wobei das Verfahren die folgenden Schritte aufweist:
Inkontaktbringen des Analysechips nach einem der Ansprüche 1 bis 6 mit einer Lösung, die eine Testsubstanz enthält, wodurch sich die Testsubstanz selektiv an die selektiv bindende Substanz bindet, die auf der Oberfläche des Trägers immobilisiert ist; und
Messen der Menge der Substanz, die durch die selektiv bindende Substanz an den Analysechip gebunden ist.

9. Verfahren zum Analysieren einer Testsubstanz nach Anspruch 8, wobei die die Testsubstanz enthaltende Lösung einer Entgasungsbehandlung unterzogen wird, bevor die die Testsubstanz enthaltende Lösung mit dem Analysechip in Kontakt gebracht wird.

## Revendications

1. Puce d'analyse comprenant :
un substrat qui présente une surface sur laquelle sont immobilisées une ou des substances de liaison sélectives ;
un élément de couverture collé sur ledit substrat;
un vide situé entre ledit substrat et ledit élément de couverture ; et
des particules mobiles contenues ou injectées dans ledit vide ;
les surfaces desdites particules étant revêtues d'un ou d'agents tensio-actifs.

2. Puce d'analyse selon la revendication 1, dans laquelle ledit agent tensio-actif revêtu sur lesdites surfaces desdites particules est un agent tensio-actif anionique ou un agent tensio-actif non ionique.

3. Puce d'analyse selon la revendication 1 ou 2, dans laquelle ledit substrat comprend une région irrégulière composée de parties en retrait et de parties en saillie, et ladite ou lesdites substances de liaison sélectives sont immobilisées sur les surfaces supérieures desdites parties en saillie.

4. Puce d'analyse selon l'une quelconque des revendications 1 à 3, dans laquelle le matériau qui constitue lesdites particules revêtues avec ledit ou lesdits agents tensio-actifs comprend une céramique.

5. Puce d'analyse selon l'une quelconque des revendications 1 à 4, dans laquelle un ou plusieurs trous de pénétration qui communiquent avec ledit vide sont formés dans ledit élément de couverture.

6. Puce d'analyse selon l'une quelconque des revendications 1 à 5, dans laquelle la distance la plus courte entre ladite surface dudit substrat, sur laquelle sont immobilisées ladite ou lesdites substances de liaison sélectives, et ledit élément de couverture, est plus petite que le diamètre desdites particules.

7. Puce d'analyse selon l'une quelconque des revendications 1 à 6, dans laquelle ladite substance de liaison sélective est un ADN, un ARN, une protéine, un peptide, un saccharide, une chaîne de sucre ou un lipide.

8. Procédé destiné à analyser une substance d'essai, ledit procédé comprenant les étapes consistant à :
mettre en contact ladite puce d'analyse selon l'une quelconque des revendications 1 à 6 avec une solution contenant une substance d'essai, en liant de ce fait de manière sélective ladite substance d'essai à ladite substance de liaison sélective immobilisée sur la surface dudit substrat ; et
mesurer la quantité de ladite substance liée à ladite puce d'analyse par l'intermédiaire de ladite substance de liaison sélective.

9. Procédé destiné à analyser une substance d'essai, selon la revendication 8, dans lequel ladite solution contenant ladite substance d'essai est soumise à un traitement de dégazage avant de mettre en contact ladite solution qui contient ladite substance d'essai avec ladite puce d'analyse.
